(19) Europäisches Patentamt
European Patent Office
Office européen des brevets

(11) EP 2 308 847 B1

(12) **EUROPEAN PATENT SPECIFICATION**

(45) Date of publication and mention of the grant of the patent:
**02.04.2014 Bulletin 2014/14**

(21) Application number: **09012781.2**

(22) Date of filing: **09.10.2009**

(51) Int Cl.:
***C07D 213/79*** *(2006.01)* ***C07D 213/81*** *(2006.01)*
***C07D 413/12*** *(2006.01)* ***A61K 31/4418*** *(2006.01)*
***A61K 31/4427*** *(2006.01)* ***A61P 3/10*** *(2006.01)*

(54) **Substituted pyridines as inhibitors of dipeptidyl peptidase IV and their application for the treatment of diabetes and related diseases**

Substituierte Pyridine als Inhibitoren der Dipeptidyl Peptidase IV und ihre Anwendung für die Behandlung von Diabetes und verwandten Krankheiten

Pyridines substituées servant d'inhibiteurs de dipeptidyl-peptidase IV et leurs application pour le traitement du diabète et des maladies associées

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**13.04.2011 Bulletin 2011/15**

(73) Proprietor: **EMC microcollections GmbH**
**72070 Tübingen (DE)**

(72) Inventors:
- **Schaffner, Arnaud-Pierre, Dr.**
  **72108 Rottenburg (DE)**
- **Kaczanowska, Katarzyna, Dr.**
  **72070 Tübingen (DE)**
- **Bächle, Daniel, Dr.**
  **72768 Reutlingen (DE)**

(56) References cited:
**WO-A-2005/042488** **WO-A-2006/090915**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 308 847 B1

## Description

Field of the invention

The present invention relates to novel 4-aryl substituted pyridines, which are selective and highly potent inhibitors of the dipeptidyl peptidase IV enzyme, and which are useful in the treatment and prevention of multiple diseases and disorders mediated by dipeptidyl peptidase IV, such as diabetes, particularly type II diabetes.

The present invention also relates to specially developed procedures for synthesis of such compounds and their analogs as well as to pharmaceutical compositions comprising these compounds in combination with other therapeutic agents.

The use of these compounds and compositions as medicament in the prevention and treatment of diseases or disorders in which the dipeptidyl peptidase IV enzyme is involved, is also disclosed as well as their use for diagnostic purposes.

Background of the invention

Dipeptidyl peptidase IV (DPP-IV) is a highly specific atypical serine type protease, which specifically cleaves dipeptides from proteins and oligopeptides after a penultimate N-terminal proline or alanine. This enzyme was described in 1966 for the first time (V.K.Hopsu-Hav and G.G.Glenner, Histochem. 1966, 7, 197-201). Only over the years it was detected, that this enzyme plays an essential role as physiological regulator within the mammalian organism.

DPP-IV as a complex enzyme is released in almost all tissues and organs. It is expressed on the surface of most cell types and can also be found in plasma and urine. By its important modulatory activity on a number of chemokines, neuropeptides, and peptide hormones it is associated, among other processes, with immune regulation, signal transduction, and apoptosis (D.J.Drucker, Diabetes Care 2007, 30(6), 1335-1343; S.Lorey et al., J. Biol. Chem. 2002, 277(36), 33170-33177).

In a healthy organism pancreatic $\alpha$ cells of the islets of Langerhans release glucagon to stimulate hepatic glucose production when the level of blood sugar is low, while $\beta$ cells of the islets of Langerhans in the pancreas produce insulin when it is high. Additionally, in response to nutrient ingestion, several hormones are released from the gastrointestinal tract to stimulate glucose dependent insulin biosynthesis and secretion. This so called incretin-effect is mediated by the glucose dependent insulinotropic peptide (GIP), which is expressed in the K cells of the duodenum and by the glucagon-like peptide 1 (GLP-1), which is expressed in the L cells of the mucosa in distal small intestine.

In type II diabetes however, caused by a combination of insulin resistance and ß cell dysfunction, the normal incretin response is markedly reduced. ß cells are under-active and reduced in number, insulin secretion is lower, and insulin mediated inhibition to $\alpha$ cells is diminished. As a result the glucagon-insulin balance is lost. So the $\alpha$ cells are highly overactive, stimulating the liver to produce further glucose and resulting in both fasting and postprandial hyperglycemia. Consequently diabetes is associated with an increased and premature risk of specific macro and microvascular, preferably cardiovascular, complications and other diseases. Often diabetes patients suffer from obesity.

A higher activity of the incretin hormones however should result in an increase of the insulin secretion and counterbalance the problems in non-insulin dependent diabetes. It is known that DPP-IV is responsible for rapid inactivation of the incretins by cleavage. But because of the short *in vivo* half-life of about 1.5 min (B.D.Green et al., Diab.Vasc.Dis.Res. 2006, 3(3), 159-165), it will be not useful to applicate the GLP-1 as it is. That's why the synthesis of DPP-IV resistant incretin mimetics like exenatide was a promising approach (Eli Lilly & Co., US 5,424,286). But these therapeutic agents as well as synthetic GLP-1 have to be administered parenterally, cause some side effects and are only approved in combination with other oral anti-diabetic agents.

Another completely new therapy approach regarding the incretin-effect, the inhibition of DPP-IV, has been shown to be able to prolong the beneficial effects of primarily GLP-1 and GIP by stabilizing the intact (active) form of the hormones (I.Quesada et al., J.Endocrin. 2008, 199, 5-19). The longer activity of the incretins like GLP-1 permits an increased secretion of insulin and inhibits the glucagon release, which in turn decreases the chronically high blood glucose to near-normal levels, shown by $HbA_{1c}$ values. It could also be proven that a higher GLP-1 level prevents the remaining pancreatic ß cells, increases their activity and promotes their growth resulting in an increasing number and mass of ß cells. By slowing the rate of gastric emptying and promoting the satiety, GLP-1 is also useful for reduction of the caloric intake and in this turn for weight reduction (A.H.Barnett, Clin.Endocrin. 2009, 70, 343-353). Since the activity of incretin hormones depends on the blood glucose level, there is a lower risk of hypoglycemia, compared to conventional anti-diabetic agents.

Owing the impressive anti-diabetic actions of GLP-1, targeting both fasting and prandial glucose concentrations, an effective inhibition of DPP-IV will be a new promising pathway for treatment of diabetes, particularly non-insulin dependent diabetes and related diseases. Since GLP-1 inhibits the glucagon release also, inhibitors of DPP-IV should also be usable for the treatment of type I diabetes.

These facts led to an elevated interest in the inhibition of DPP-IV enzyme for treatment of diabetes (B.Kuhn et al., Current Topics in Medicinal Chemistry 2007, 7, 609-619).

From 1996, when the first *in vivo* experiments indicated that DPP-IV inhibition results in improved glucose tolerance, a number of pharmaceutical companies announced an interest in the application of DPP-IV inhibitors in the treatment of type II diabetes.

Extensive research efforts from both the academia and the pharmaceutical industry have led to launch of Sitagliptin (2006, Merck & Co. Inc., WO 2003/004498) and Vildagliptin (2007, Novartis AG, WO 2000/034241, in Europe only) and the advancement of a few others into preregistration or phase III, for example, Saxagliptin (Bristol-Myers Squibb Co., WO 2001/068603), Alogliptin (Takeda, WO 2005/095381) and ABT-279 (Abbott Laboratories, US7,238,724) (S.H.Havale and M.Pal, Bioorg.Med.Chem. 2009, 17, 1783-1802). In the most cases the administration of these compounds together with a second anti-diabetic agent is recommended (B.T.Srinivasan et al., Postgrad.Med.J. 2008, 84, 524-531).

Nevertheless continuing search for small molecules as potent and selective orally available inhibitors remains a major challenge and is indicated by an explosion of patents and publications regarding a big variety of scaffolds, in particular from pharmaceutical industry. A lot of efforts were made to prolong the stability and the long-acting potential of conventional DPP-IV inhibitors (H.Fukushima etal., Bioorg.Med.Chem. 2008, 16, 4093-4106; H.Fukushima etal., Chem.Pharm.Bull. 2008, 56(8), 1110-1117). Also some approaches were made on the base of crystal structure analysis by target-oriented design (T.Lübbers et al., Bioorg.Med.Chem.Lett. 2007, 17, 2966-2970). A comprehensive review about the recent approaches in medicinal chemistry to the inhibition of DPP-IV for treatment of type II diabetes is given by Havale and Pal (S.H.Havale and M.Pal, Bioorg.Med.Chem. 2009, 17, 1783-1802).

Recently several compounds based on the pyridine backbone have been reported, which show a highly effective peptidase inhibitory activity and should be useful as an agent for prophylaxis and treatment of diabetes and further DPP-IV mediated diseases.

In 2003, F.Hoffmann-La RocheAG (WO 2003/068757) reported compounds of formula (Y), wherein X is N or C-$R^5$, $R^1$ and $R^2$ are independently hydrogen or lower alkyl, $R^3$ is heterocycle or aryl, $R^4$ is lower alkyl, alkoxy, alkylthio, substituted or not substituted heterocyclyl or aryl, and $R^5$ is hydrogen or lower alkyl.

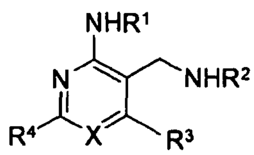

(Y)

However, this patent does not report any compound of the present invention due to different substitution pattern especially on the positions 2 (amine group $NHR^1$) and 6 of the pyridine-ring ($R^4$).

In 2005, Takeda Pharmaceutical Company (WO 2005/042488) reported pyridin compounds of formula (YY), wherein $R^1$ and $R^2$ are each independently optionally substituted hydrocarbon or hydroxy groups, $R^3$ is an optionally substituted aromatic group, $R^4$ is an optionally substituted amino group, L is a divalent chain hydrocarbon group, Q is a bond or a divalent chain hydrocarbon group, and X is a hydrogen atom, a cyano group, a nitro group, an acyl group, a substituted hydroxy group, an optionally substituted thiol group, an optionally substituted amino group or an optionally substituted cyclic group, or a salt thereof.

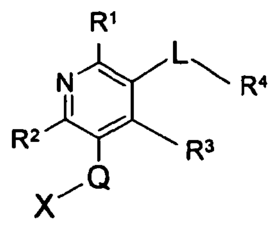

(YY)

Even though this patent represents the closest prior art, it does not report any compound of the present invention due to a different substitution pattern especially on position 6 ($R^2$) of the pyridine-ring limited to alkyl groups and in the order of the substituents on positions 5 and 6.

One year later, Takeda Pharmaceutical Company (WO 2006/090915) specified the previous invention and

reported pyridyl acetic acid compounds of formula (YYY), wherein $R^1$ is an alkyl group optionally substituted by a cycloalkyl group, $R^2$ is an alkyl group, $R^3$ is a hydrogen, an alkyl group or a halogen atom, and X is $-OR^6$ or $-NR^4R^5$ wherein $R^4$ and $R^6$ are each independently a hydrogen atom, an optionally substituted hydrocarbon group or an optionally substituted heterocyclic group, $R^5$ is an optionally substituted hydrocarbon group, an optionally substituted heterocyclic group or an optionally substituted hydroxy group, or $R^4$ and $R^5$ optionally form, together with the adjacent nitrogen atom, an optionally substituted nitrogen-containing heterocycle, or a salt thereof.

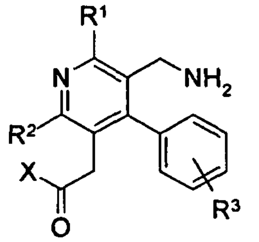

(YYY)

However, this patent does not report any compound of the present invention due to a different substitution pattern especially on position 6 ($R^2$) of the pyridine-ring limited to alkyl groups, with the substitution on position 5, wherein the carboxylic or amide group is bound via a methylene, and in the order of the substituents on positions 5 and 6.

The same year, Bristol-Myers Squibb Company (WO 2006/127287) reported pyrrolopyridine-based inhibitors of DPP-IV and methods to synthesize them.

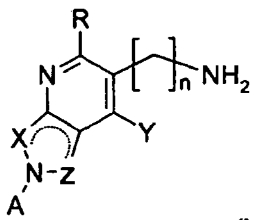

(YYYY)

These compounds are provided having the formula (YYYY) wherein "... " represents one or two double bonds in the 5-membered ring, and the six-membered ring is an aromatic ring, n is 1 or 2, R is a functional group selected from the group consisting of hydrogen, halogen, cyano, amino, $CF_3$, alkyl, aryl, X and Z are the same or different and are independently selected from the group consisting of $CH_2$, CH, C=O, $C=CR^3R^4$, $CR^3R^4$, $C=NR^3$, and C=S, wherein $R^3$ and $R^4$ are alkyl or aryl, Y is aryl or heteroaryl and A is a functional group selected from the group consisting of hydrogen, alkyl, aryl, $O-R^1$, cyano, amino, -C(O)-OH, $-C(O)-NR^1R^2$, $-C(O)-OR^1$, $-NR^1R^2$, $-NR^1-C(O)R^2$, $-NR-SO_2R^2$, $S(O)_m-R^1$, and $-S(O)_2NR^1R^2$.

Most recently, Bristol-Myers Squibb Company extended the previous invention to 6-membered rings (WO 2008/064107) according to formula (YYYYY).

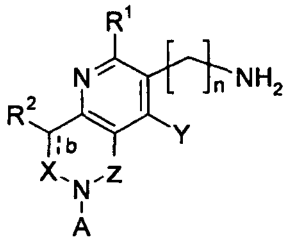

(YYYYY)

However, these patents also do not report any compound of the present invention due to major differences in the substitutions onto the pyridine-ring on positions 5 and 6, especially with the presence of the five or six-membered rings as substituents.

Also other substituted pyridine- as well as related pyrimidine-containing compounds were taken into account as DPP-IV inhibitors. Examples of potent inhibitors from this class are aminomethylpyrimidines identified at F.Hoffmann-LaRocheAG (J.U.Peters et al., Bioorg. Med. Chem.Lett. 2004, 14, 3575-3578). KR-62436 (Korea Research Institute of

Chemical Technology), a potent DPP-IV inhibitor with an excellent oral bioavailability, was progressed into clinical trials, but discontinued due to its side effects (T.E.Hughes et al., Biochemistry 1999, 38, 11597-11603). Mentioned above pyridine containing ABT-279, a compound with excellent potency and selectivity for DPP-IV inhibition is presently in phase III clinical trials (D.J.Madar etal., J.Med.Chem. 2006, 49, 6416-6420).

Although obviously some pyridine containing compounds have been proven as DPP-IV inhibitors, there is no report on the compounds of the present invention. Also the substitution on position 6 of the pyridine-ring by an aryl or heteroaryl was never reported before. The presented novel 4-aryl substituted pyridines, bearing primary amine and a simple substitution pattern, constitute original molecules with a highly selective DPP-IV inhibitory potency.

Many DPP-IV inhibitors were developed before the discussion on potential toxicity due to the inhibition of other members of DPP family resulting in unforeseen side effects and low tolerance.

In this context especially two closely related proteases, DPP-8 and -9, were recently intensively investigated. *In vivo* experiments, reported by Lankas et al., showed that inhibition of DPP-8 and -9 is associated with severe toxicity in rats and that it provokes attenuation of T-cell proliferation in human *in vitro* models (G.R.Lankas et al., Diabetes 2007, 56, A138). Additionally DPP-8 and -9 are reported to be up-regulated in experimentally induced asthma (J.Schade et al., J.Histochem.Cytochem. 2008, 56, 147-155).

So it has become necessary to design more selective inhibitors targeting DPP-IV Especially DPP-8, a cytoplasmic protease, shares 51% amino acid similarity with the protein sequences of DPP-IV The high degree of sequence homology between DPP-8, -9 and DPP-IV makes selective inhibitor design a challenging task (P.VanderVeken et al., Current Topics in Med.Chem. 2007, 7, 621-635; P.Van der Veken et al., Bioorg.Med.Chem. Lett. 2008, 18, 4154-4158).

Summary of the invention

In view of the prior art described above and the expected rapid increase of the number of patients suffering from diabetes, there is a considerable demand for the development of new compounds having a highly selective peptidase inhibitory action. These compounds should be useful preferably as orally deliverable agents, for the prophylaxis and treatment of diseases and maladies in which the dipeptidyl peptidase IV enzyme is involved, especially for the therapy of type II diabetes and related diseases.

There is particularly a need for agents with superior efficacy, long duration of action, high selectivity and good physiological compatibility, which avoid the mentioned drawbacks of the conventional therapies like decreasing long-term effects, need of a second medication, need of multiple application, immunosuppressive effects, increased risk of infection, and other considerable side effects (C.W.Chia and J.M.Egan, J.Clin.Endocrinol.Metab. 2008, 93(10), 3703-3716; S.Lorey etal., Eur.J.Biochem. 2003, 270, 2147-2156).

The provided novel 4-aryl substituted pyridines with their demonstrated highly effective and selective DPP-IV inhibitory activity exactly represent this type of therapeutic compound, demanded.

In accordance with the present invention, compounds of formula (I) and salts or solvates thereof are provided.

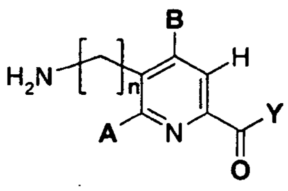

**(I)**

Formula (I) is characterized by a chemical structure wherein an amino group is bonded on the 3-position of the pyridine-ring via an alkyl group, an optionally substituted aryl group is bonded on the 4-position, a hydrogen is bonded on the 5-position, and an acyl group is bonded on the 6-position.

The inventive compounds are characterized by a superior peptidase inhibitory action and a high selectivity of their biological activity. Therefore they are useful as new agents for the prophylaxis and treatment of conditions mediated by DPP-IV, particularly diabetes and related diseases.

Accordingly, the present invention relates to

1) compounds represented by formula (I) and any salts or solvates thereof, as described in claim 1 (preferred embodiments of these compounds are described in claim 2),

2) methods of producing compounds represented by formula (I) and any salts or solvates thereof, as described in

claim 3,

3) pharmaceutical compositions comprising a compound according to formula (I), any salts or solvates thereof, and optionally further therapeutic agents, which are useful for the prophylaxis, treatment or delaying of the progression and onset of diabetes, diabetic complications, impaired glucose tolerance or obesity, related diseases, and other conditions mediated by DPP-IV as described in claims 4 and 5,

4) use of the compounds (I) and salts or solvates thereof for the production of a diagnostic kit for clinical applications and scientific research, as described in claim 6,

5) use of the compounds (I) and salts or solvates thereof for the production of an agent for the prophylaxis, treatment or delaying of the progression and onset of diabetes, diabetic complications, impaired glucose tolerance or obesity, related diseases and other conditions mediated by DPP-IV as described in claim 7,

6) use of the compounds (I) and salts or solvates thereof for the production of a peptidase inhibiting agent, medicament or pharmaceutical composition, wherein the peptidase is DPP-IV, for preventing, treating or delaying of the progression or onset of diabetes, diabetic complications, impaired glucose tolerance, obesity, related diseases and other conditions mediated by DPP-IV in a mammal, as described in claim 8,

Hereby the original terms of all claims are declared to be content of the description of the invention by reference.

Detailed description of the invention

Hereinafter, the best mode for carrying out the present invention and preferred embodiments are described in detail.

The presented invention relates to novel 4-aryl substituted pyridine compounds with the following general structure

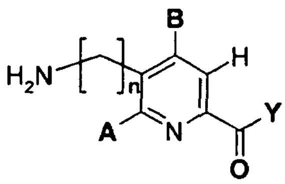

(I)

wherein

| | |
|---|---|
| n | is 1 or 2; |
| A | is alkyl consisting of 1 to 6 C-atoms; |
| B | is aryl, which may optionally be substituted with one to five substituents selected from the group consisting of hydrogen (H), halogen, $CF_3$, alkyl, and alkoxy; |
| Y | is selected from the group consisting of hydroxy, alkoxy, and $-NR^3R^4$, wherein |
| $R^3$ and $R^4$ | are the same or different and are |

(i) each independently selected from the group consisting of hydrogen (H), alkyl, cycloalkyl or saturated and unsaturated heterocycloalkyl, wherein either functional group may optionally be substituted with one to three substituents selected from the group consisting of hydrogen (H), alkyl, alkylester, alkylamino, heterocycloalkyl, hetero-cycloalkylamino, substituted or unsubstituted aryl, amino, and cyano;
(ii) $R^3$ and $R^4$ in $-NR^3R^4$ form together with the adjacent nitrogen atom a 3- to 6-membered saturated or partially unsaturated heterocycloalkyl ring system, which may optionally be substituted with one to three substituents selected from the group consisting of hydrogen (H), alkyl, alkylester, alkylamino, heterocycloalkyl, heterocyclo-alkylamino, substituted or unsubstituted aryl, amino, and cyano.

As preferred embodiments relating to the compounds of the invention, but not limiting to, can be mentioned compounds of formula (II)

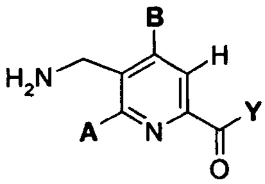

(II)

wherein the substituents are defined in the same way as for formula (I) above.

As especially preferred embodiments relating to the compounds of the invention, but not limiting to, can be mentioned the following compounds according to the chemical structures (V) to (XIV). The activity of the 2-aryl regioisomer relating to compound (XV) was characterized for comparison.

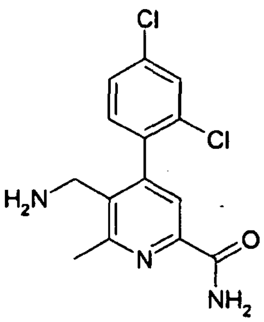

(V)

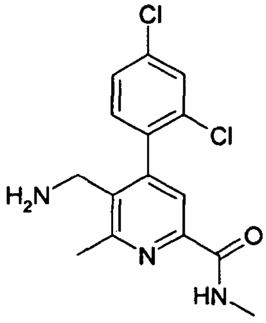

(VI)

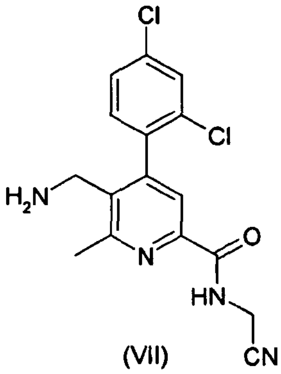

(VII)

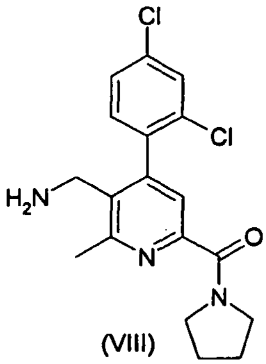

(VIII)

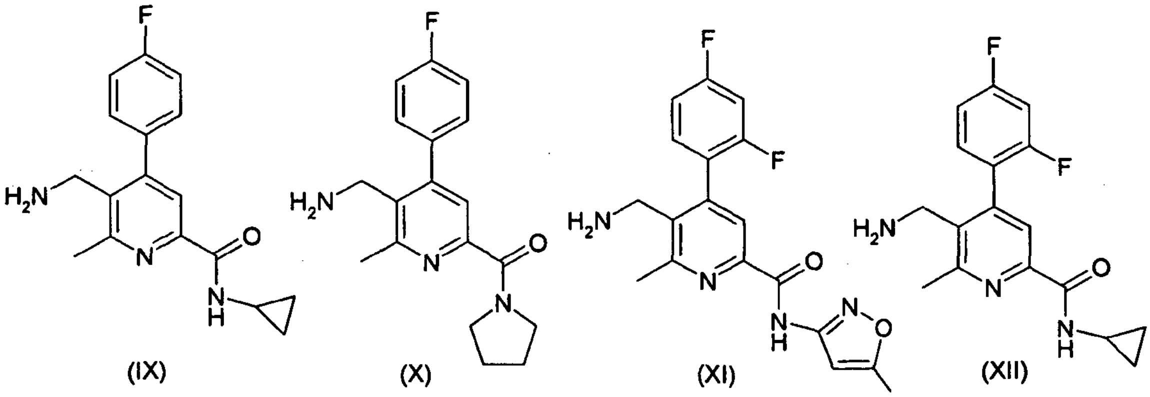

(IX) (X) (XI) (XII)

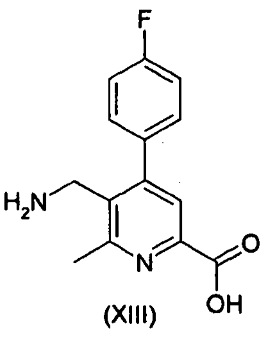

(XIII)

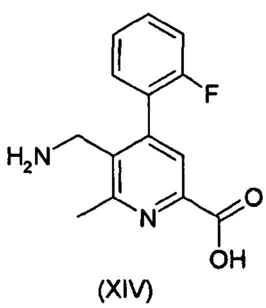

(XIV)

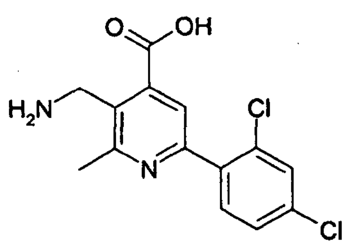

(XV)

These definitions include the free form, all inorganic or organic salts (pharmaceutically acceptable, i.e. non-toxic, physiologically acceptable salts, are preferred), solvates, and all tautomers, stereoisomers, and mixtures of the inventive compounds of formulae (I), (II), and (V) to (XIV).

The inventive compounds of formulae (I) and (II) can be prepared as shown below in the following reaction schemes and descriptions thereof, as well as by using relevant published literature procedures. Exemplary reagents and procedures for these reactions appear herein and in the working Example A. Insofar as the preparation is not particularly described herein, a compound used as starting material is known or may be prepared from known compounds in known manner or analogously to known methods.

Finally, compounds of the invention are either obtained in free form, or as a salt thereof if salt forming groups are present. As salt of the inventive compounds a pharmaceutically acceptable salt is preferable. Examples of such a salt include a salt with an inorganic or organic base, a salt with an inorganic or organic acid, a salt with a basic or acidic amino acid, and the like. The inventive compounds can also be converted in prodrug esters thereof, solvates, and the like.

Scheme 1 provides a general route to prepare pyridine amines regarding formulae (I) and (II) of the invention.

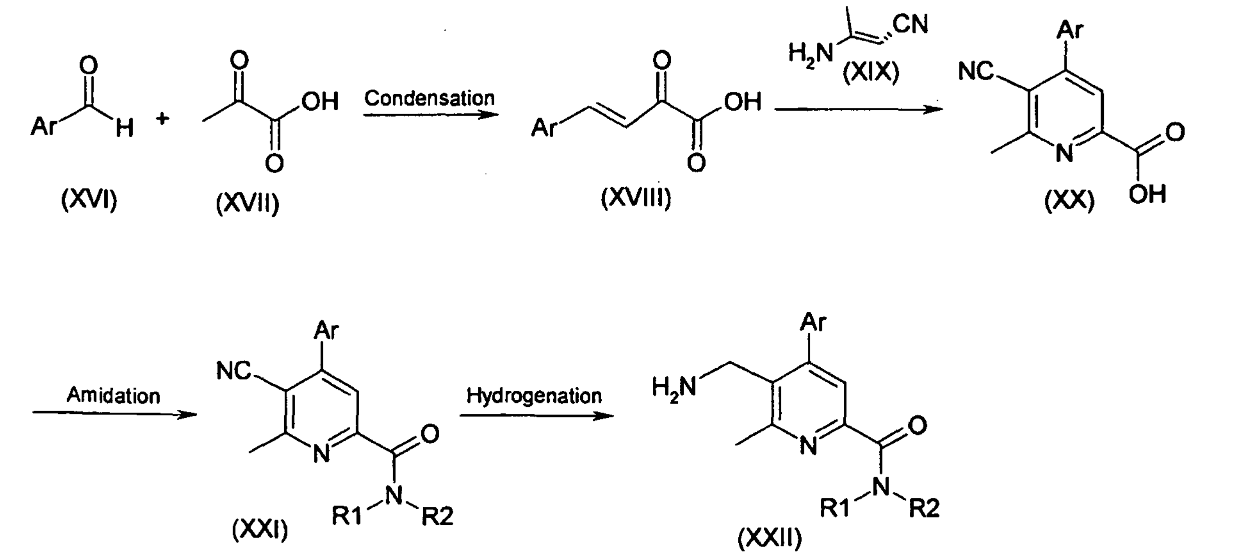

(XVI) (XVII) (XVIII) (XIX) (XX) (XXI) (XXII)

Scheme 1

Aldol condensation of commercially available aromatic aldehydes (XVI) with pyruvic acid (XVII) yields (*E*)-2-oxo-4-aryl-but-3-enoic acids (XVIII). Treatment of (XVIII) with potassium *tert*-butoxide in acetonitrile at ambient temperature affords pyridines (XX) by condensation of (XVIII) with *in situ* generated ß-aminocrotonitrile (XIX). Amides of formula (XXI) were obtained by amidation reaction. Transformation of nitrile group of (XXI) to amines (XXII) might be performed by catalytic hydrogenation.

An alternative route leading to the inventive pyridine amines of formulae (I) and (II) is given in Scheme 2.

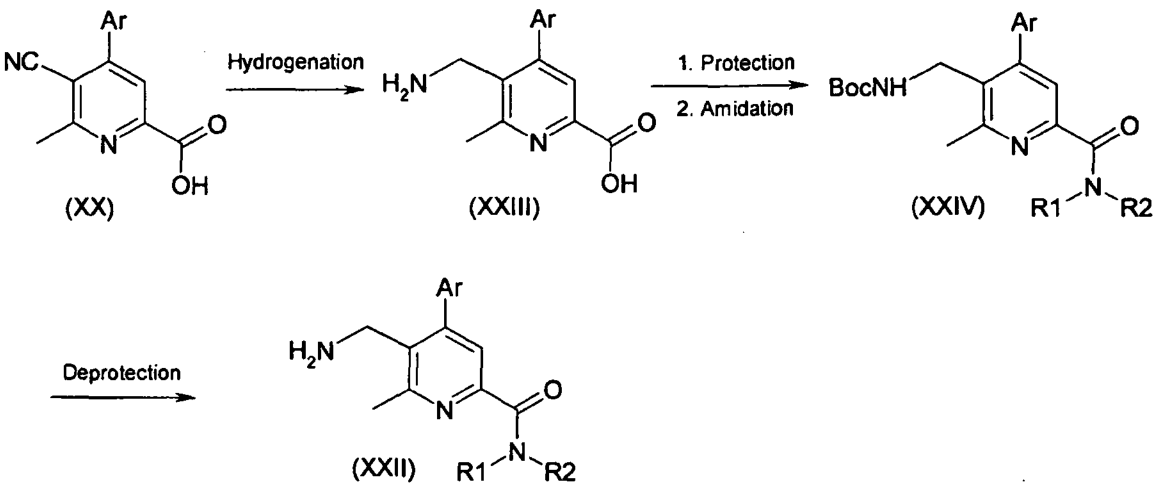


(XX) (XXIII) (XXIV) (XXII)

Scheme 2

The pyridines (XX) might be reduced first to the corresponding amines (XXIII) by catalytic hydrogenation. Protection of the amine functionality with di-*tert*-butyl dicarbonate or other protecting group known in the art, followed by amidation, affords compound (XXIV) that can be deprotected to yield (XXII).

As presented in Scheme 3, compound (XXIII) can additionally be modified by N-oxidation of the pyridine scaffold.

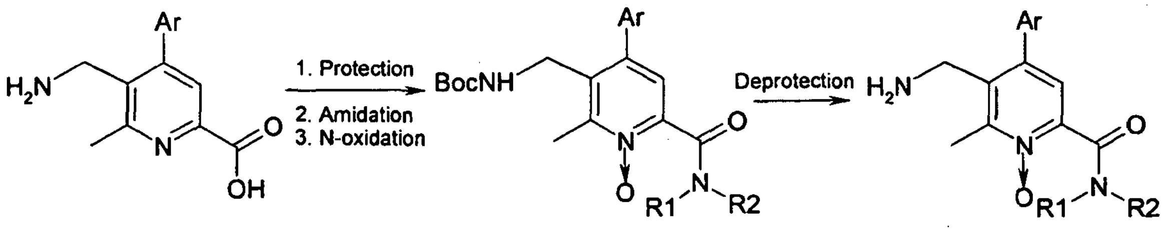


(XXIII) (XXV) (XXVI)

Scheme 3

Boc-protection of amine group followed by amidation and N-oxidation leads to compound (XXV), which after acidic deprotection yields (XXVI).

The corresponding 2-aryl regioisomers (III) and (IV) of the compounds (I) and (II)

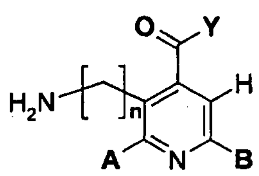

(III)

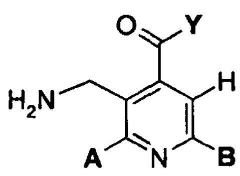

(IV)

might be synthesized via two similar routes given in Scheme 4 and Scheme 5.

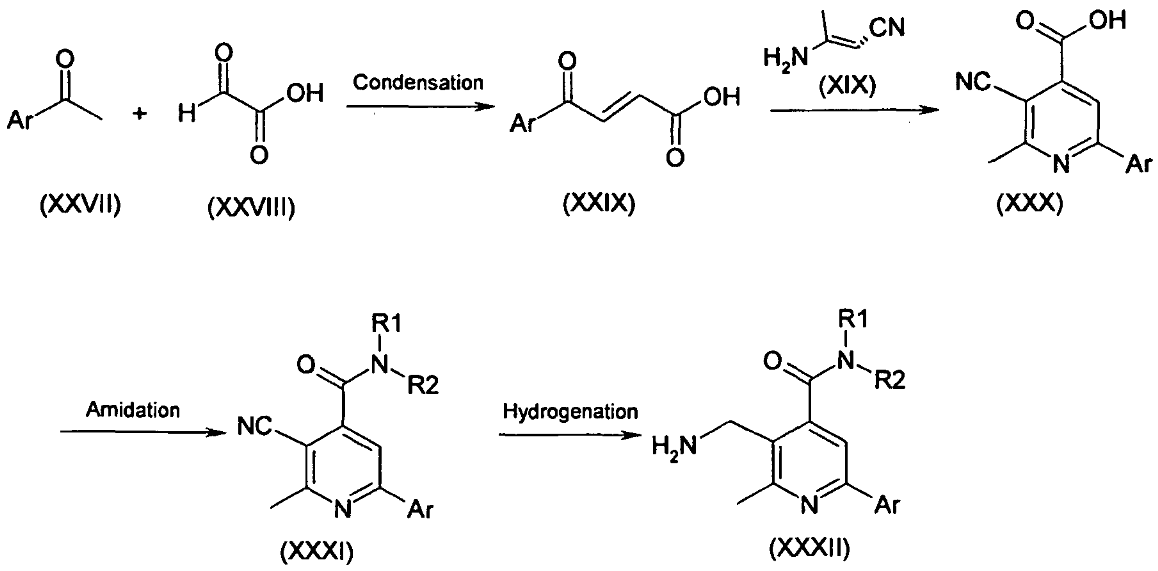

(XXVII) (XXVIII) (XXIX) (XXX)
(XXXI) (XXXII)

Scheme 4

Condensation of commercially available acetophenones (XXVII) with glyoxylic acid (XXVIII) yields (*E*)-4-oxo-4-aryl-but-2-enoic acids (XXIX), which can be reacted with *in situ* generated ß-aminocrotonitrile (XIX) affording pyridine (XXX). Conversion to the primary amine (XXXII) can be accomplished following the route similar to the one described in Scheme 1.

An alternative method of preparing pyridine amines (XXXII) is presented in Scheme 5 and corresponds to the preparation of its regioisomer (XXII) as described in Scheme 2.

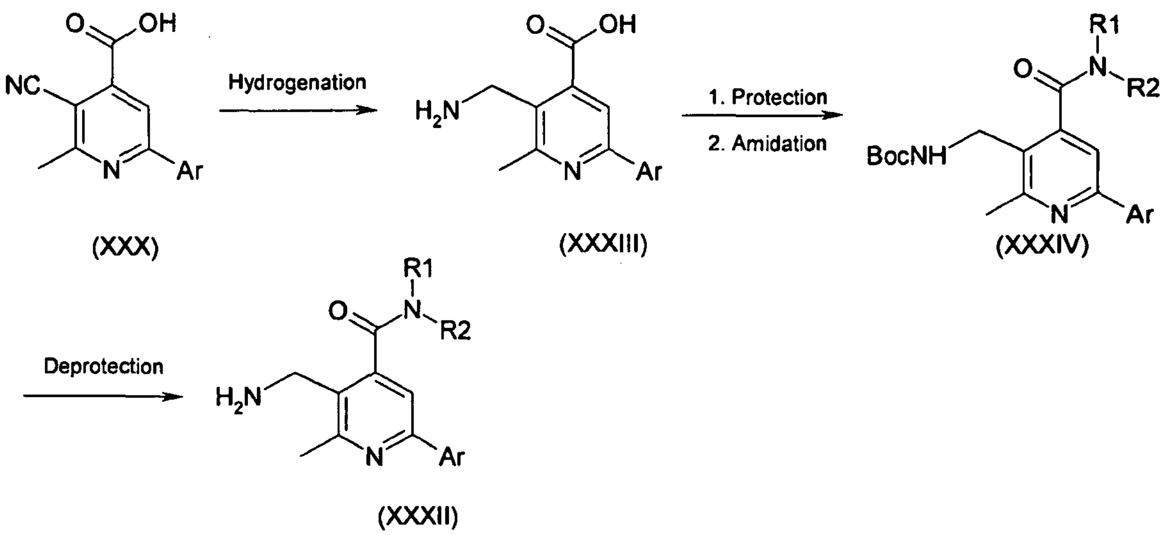

(XXX) (XXXIII) (XXXIV)
(XXXII)

Scheme 5

The DPP-IV inhibitory potency of the compounds of the invention and their corresponding salts or solvates was demonstrated employing an *in vitro* DPP-IV drug discovery assay, which measures the ability of test compounds to inhibit the activity of isolated human DPP-IV enzyme. The potency of the tested compounds is described upon the degree in inhibition of DPP-IV cleavage of the chromogenic substrate Gly-Pro-pNA (pNA is p-nitroaniline), expressed as $IC_{50}$ values.

The selectivity of the compounds of the invention was shown by an analog assay with isolated human DPP-8 and the same substrate. The implementation of both assays and some representative results regarding the compounds of the invention are described in detail in the experimental section (Example B).

*In vitro* investigations with human cervix carcinoma cells were implemented to examine for toxic effects on

human cell lines.

The applicant has surprisingly discovered that the presented inventive 4-aryl substituted pyridines exhibit precious pharmacological properties, in particular a superior inhibiting effect on DPP-IV enzyme activity. In comparison to the known DPP-IV inhibitor lie-Pro-Ile the inventive compounds have a more then 300 times higher activity. In comparison to DPP-IV inhibitors actually on the market, the $IC_{50}$ levels are in the same nanomolar range.

As shown by the inventors also, the inventive compounds are, in contrast to the actually approved DPP-IV inhibitors, highly selective at the inhibition of DPP-IV Surprisingly their potency to inhibit DPP-8 enzymes is up to 6,600 times, on average 1,000 times, lower than the DPP-IV inhibitory activity.

Furthermore the inventive compounds show an excellent safety profile with minimal side effects, low toxicity, and high stability.

It was particularly unexpected that the inventive pyridine derivatives with their simple substitution pattern can provide such an improved pharmacological profile in relation to the conventional DPP-IV inhibitors. The novel compounds show some pharmacokinetic and pharmacological advantages, e.g. less side effects, better bioavailability, long duration of action, and above all high selectivity and activity. With regard to this impressive pharmacological profile the inventive compounds represent, alternatively to the conventional approved pharmaceuticals on the market, a really effective agent for the treatment and prophylaxis of DPP-IV mediated diseases.

In view of their abilities in inhibiting DPP-IV above mentioned and of findings in the literature the compounds disclosed herein and their corresponding salts, solvates or prodrugs are expected to be useful as pharmaceuticals in the prevention, treatment or delaying of the progression and onset of diseases or maladies mediated by dipeptidyl peptidase IV enzyme, such as of diabetes, especially non-insulin dependent diabetes mellitus, more especially type II diabetes, and related diseases such as impaired glucose tolerance, impaired fasting plasma glucose, insulin resistance, insulin secretagogue, cachexia, hyperglycemia, hyperinsulinemia, hypercholesterolemia, fatty liver, elevated blood levels of fatty acids or glycerol, increasing high density lipoprotein levels, obesity, hyperlipidemia including hypertriglyceridemia, Syndrome X, dyslipidemia, acidosis, ketosis; including but not limited to complications of diabetes such as retinopathy, neuropathy, nephropathy, erectile dysfunction, cataracts, delayed wound healing, arthritis, calcitonin-osteoporosis, ischemia, arteriosclerosis, polycystic ovary syndrome, kidney disease, muscular dystrophy, myocardial infarction, angina pectoris, cerebrovascular accident, Alzheimer's disease, Parkinson's syndrome, anxiety, dementia, schizophrenia, metabolic syndrome, hyperinsulinemia-induced sensory disorder, tumor, irritable bowel syndrome, acute or chronic diarrhea, inflammatory diseases, small intestine mucous membrane trauma, malabsorption, testis function disorder, visceral obesity syndrome, hypertension, various immunomodulatory diseases; and further conditions of impaired glucose tolerance, and the like.

In addition, based on the role of GLP-1 and GLP-2 and their association with DPP-IV inhibition, it is expected that the compounds disclosed herein are useful for example to improve pancreatic ß cell function, to support regeneration of pancreatic ß cells, to produce a sedative or anxiolytic effect, to attenuate post-surgical catabolic changes and hormonal responses to stress, to reduce mortality and morbidity after myocardial infarction, or in treatment of conditions related to the above effects, which may be mediated by GLP-1 and/or GLP-2 levels.

By decreasing visceral fat, visceral fat accumulation, and appetite control, the inventive compounds and pharmaceutical compositions thereof can also be used for the cosmetic treatment of a mammal in order to effect a cosmetically beneficial loss of body weight.

In particular the inventive compounds improve the early insulin response to an oral glucose challenge and are therefore especially useful in treating non-insulin dependent diabetes mellitus and related diseases.

The present invention also relates to pharmaceutical compositions for inhibiting DPP-IV, especially for treating diabetes, more especially type II diabetes, and other diseases as set out herein. In particular, the present invention provides pharmaceutical compositions comprising, but not limited to, a therapeutically effective amount of a compound of formulae (I) or (II) or their pharmaceutically acceptable salts or solvates, alone or in combination, and/ or one or more other DPP-IV inhibitors, and/or one or more other anti-diabetic agents such as biguanides (e.g. metformin or phenformin), glucosidase inhibitors (e.g. acarbose or miglitol), insulins (including insulin secretagogues or insulin sentisizers), meglitinides (e.g. repaglinide), sulfonylureas (e.g. glimepiride, glibenclamide, glyburide, gliclazide, chlorpropamide, and glipizide), thiazolidinediones (glitazone, troglitazone, pioglitazone, rosiglitazone), PPAR-agonists, glycogen phosphorylase inhibitors, inhibitors of fatty acid binding protein, GLP-1 or other agonists of the GLP-1 receptor, STLT2 inhibitors, and/ or one or more anti-hyperglycemic agents, and/or one or more hypolipidemic/ lipid-lowering agents, and/or one or more anti-obesity agents, and/or one or more appetite suppressants, and/or one or more other therapeutic agents for diabetic complications such as anti-hypertensive agents, anti-platelet agents, anti-arteriosclerosis agents, therapeutic agents of osteoporosis, diuretic agents, agents for improving erectile dysfunction, therapeutic agents for incontinentia or pollakiuria, therapeutic agents for dysurea, chemotherapeutic agents, immunotherapeutic agents, anti-thrombotic agents, anti-dementia, agonists for peroxisome proliferator-activator receptors, and the like. The inventive pharmaceutical compositions may optionally comprise also one or more other conventional pharmaceutically active agents, adjuvants or materials, and/or one or more pharmaceutically acceptable carriers, excipients or diluents.

Pharmaceutically acceptable materials are, but not limited to, substances conventionally used in pharmacy and nutrition technology, particularly substances, which are listed in the appropriate pharmacopeia and which are not opposed to a physiological application.

The present invention also relates to the use of the inventive compounds and their pharmaceutically acceptable salts or solvates for the manufacture of medicaments for prevention, treatment or delaying of the progression or onset of diseases or conditions mediated by DPP-IV.

For solid preparations such medicaments are comprising, but not limited to, a therapeutically effective amount of the inventive compounds in conjunction or admixture with diluents, lubricants, binders, disintegrants, absorbants, and the like.

Injectable compositions are preferably aqueous isotonic solutions or suspensions comprising, but not limited to, a therapeutically effective amount of the inventive compounds in conjunction or admixture with solvents, dissolution aids, suspending agents, isotonic agents, buffers, soothing agents, and the like.

Suppositories are advantageously prepared from fatty emulsions or suspensions.

Said pharmaceutical compositions may be sterilized and may optionally contain an additive such as preserving, stabilizing, wetting or emulsifying agents, solution promoters, salts for regulating the osmotic pressure, buffers, and the like. In addition they may also contain other therapeutically valuable substances.

The compositions may be prepared by a conventionally used method in the field of pharmaceutical preparation, as known to a person skilled in the art, such as described in the appropriate pharmacopeia, like mixing, granulating, and coating methods.

In a further embodiment the invention relates to the use of the inventive compounds for inhibiting DPP-IV, respectively treating conditions mediated by DPP-IV, comprising administering to a mammal, e.g. a human, in need of such treatment, a therapeutically effective amount of a compound of the invention, a pharmaceutically acceptable salt or solvate thereof or a pharmaceutical composition comprising a therapeutically effective amount of a compound of the invention.

The inventive compounds of the formulae (I) and (II), respectively the inventive pharmaceutical compositions, can be administered for any of the uses described herein by any suitable means, for example orally, such as in form of tablets, capsules, caplets, granules, powders, troches, syrups, emulsions, suspensions; sublingually; bucally; parenterally such as by injection or infusion; nasally, including by inhalation; ophthalmically; topically such as in form of a cream, ointment or a transdermal patch; or rectally such as in form of suppositories; preferred as an oral formulation.

The compounds and pharmaceutical compositions of the invention can be used alone, in combination with other compounds of the present invention, or in combination with one or more other therapeutic agents active in the therapeutic areas described herein. The timing of administration is not limited. A compound of the present invention may be administered either simultaneously, before or after the other active ingredients, either separately by the same or different route of administration or together in the same pharmaceutical formulation.

The precise therapeutically effective amount for a subject to be employed for treating conditions mediated by DPP-IV will depend upon several factors including subject's size and health, nature and extent of the condition being treated, the mode of administration, the particular compound employed and the combination of therapeutics selected for administration. Thus, it is not useful to specify an exact amount in advance.

However, in general DPP-IV mediated conditions described above can be effectively treated by e.g. enteral, transdermal or parenteral administration of the inventive compounds or their salts, solvates or prodrugs in daily dosages of 0.01-10 mg/kg one to three times a day.

In view of the outstanding DPP-IV inhibitory activity of the presented compounds the invention also relates to the use of the novel compounds for the assembly of test kits in medicine and scientific research. In medicine the inventive compounds can be used for diagnostic purposes in physiological diseases, which could be related to DPP-IV. In the latter case the inventive compounds are well suited as highly active and selective standard materials for robust screening assays.

As described above in detail, the inventive novel 4-aryl substituted pyridines have the following advantages, which make them to effective alternative agents in the prevention, treatment or delaying of the progression and onset of diseases or maladies mediated by dipeptidyl peptidase IV enzyme, particularly diabetes and related diseases.

1) highly effective DPP-IV inhibitory activity,
2) high selectivity to DPP-IV,
3) good bioavailability,
4) better long term activity,
5) high stability,
6) good physiological compatibility with less side effects and low toxicity.

Full particulars regarding preferred embodiments and further features as well as benefits of the invention can

be seen from the following description of execution examples in correlation with the additional claims. The individual features mentioned above and described below are claimed at any one time independently and in any combination together.

Examples

Hereinafter, the present invention is described in more detail and specifically. These examples, which set forth the best mode presently contemplated for carrying out the invention, are intended to illustrate but not to limit the present invention.

In general, the compounds of the invention, such as the particular compounds disclosed in the following examples, have been identified to suppress the catalytic activity of dipeptidyl peptidase IV at nanomolar concentration range, thereby demonstrating that they posses utility as effective inhibitors of dipeptidyl peptidase IV. Potencies can be calculated as inhibition constants ($K_i$) or as $IC_{50}$ (inhibitory concentration 50%) and refer to the activity measured using the *in vitro* system described herein.

Example A - Synthesis of the inventive compounds

General

Starting materials (*E*)-2-oxo-4-aryl-but-3-enoic acids (XVIII) and (*E*)-4-oxo-4-aryl-but-2-enoic acids (XXIX) were synthesized according to the literature procedures (Y.-C.Wu et al. J.Org.Chem. 2006, 71, 6592; R.Messer et al. J.Org.Chem. 2004, 69, 8558).

All chemicals were used as purchased from commercial suppliers.

Analytical TLC was performed with ALUGRAM silica gel 60 F254 plates. Visualization was accomplished using UV light.

Column chromatography was carried out using MN silica gel 60 (70-230 mesh ASTM).

The mass spectra were measured on Waters LC/MS system (Alliance 2795 HPLC, SQD mass detector, PDA 996 detector; Grom-Sil 80 ODS-7 PH 4 μm, 2.0 x 40 mm; ionizing voltage 10 eV).

NMR analyses were performed on Bruker 400 Ultra Shield.

EXAMPLE 1 - Synthesis of 5-aminomethyl-4-(2,4-dichloro-phenyl)-6-methyl-pyridine-2-carboxylic acid methylamide (VI / XXIIa)

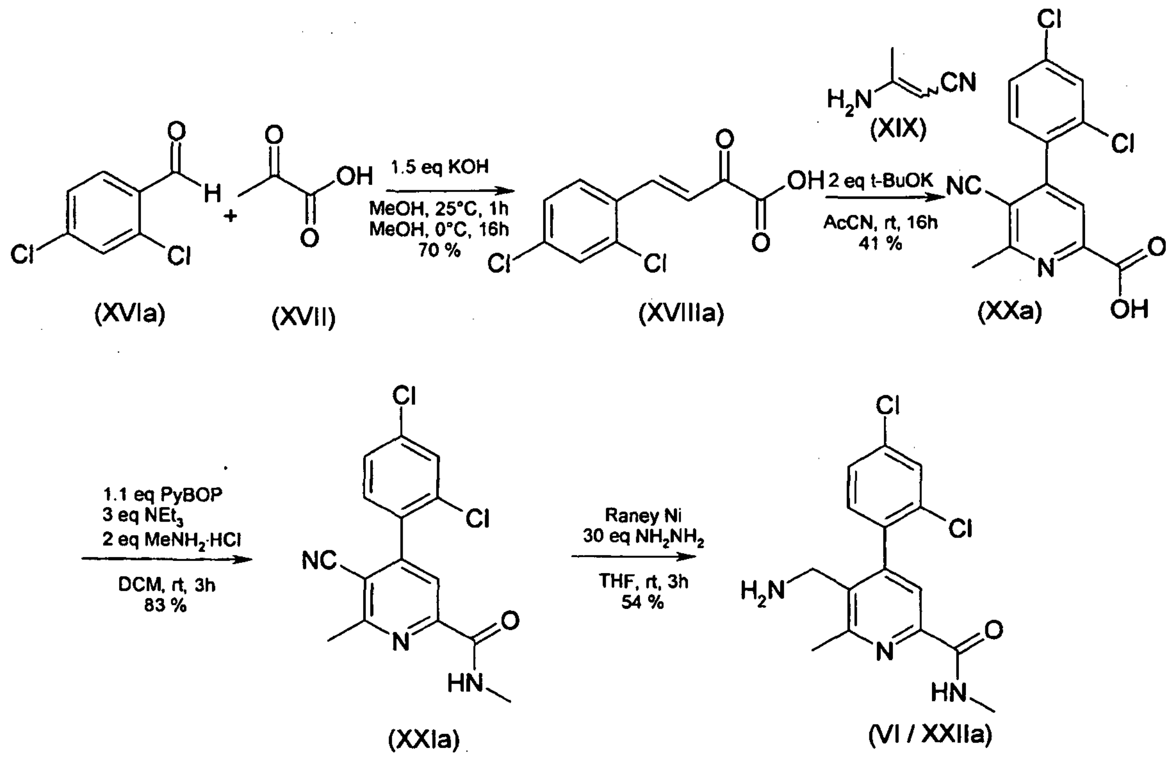


(XVIa) (XVII) (XVIIIa) (XIX) (XXa) (XXIa) (VI / XXIIa)

Step 1A. (*E*)-2-oxo-4-(2,4-dichloro-phenyl)-but-3-enoic acid (XVIIIa)

A solution of potassium hydroxide (1.5 eq, 45 mmol, 2.52 g) in methanol (7.5 mL) was added dropwise to a solution of pyruvic acid (XVII) (1 eq, 30 mmol, 2.09 mL) and 2,4-di-chlorobenzaldehyde (XVIa) (1 eq, 30 mmol, 3.50 g) in methanol (15 mL) stirred in an ice bath. The reaction temperature was kept 1 h at ~25°C and at 0°C overnight. The yellow solid was filtered off and washed twice with cold methanol and once with diethyl ether. The crude residue was taken up in water, acidified with 1 M HCl and extracted with DCM. The combined organic phases were dried over anhydrous sodium sulphate and concentrated to afford the product, a yellow solid (5.15 g, 70%), that was used without further purification.

Step 1B. 5-Cyano-4-(2,4-dichloro-phenyl)-6-methyl-pyridine-2-carboxylic acid (XXa)

Potassium *tert*-butoxide (2 eq, 40 mmol, 4.49 g) was added portionwise to a solution of (*E*)-2-oxo-4-(2,4-dichloro-phenyl)-but-3-enoic acid (XVIIIa) (1 eq, 20 mmol, 4.90 g) in acetonitrile (100 mL) stirred at room temperature. After 16 h (reaction monitored by LC/MS) the solvent was removed under reduced pressure. The crude product was taken up in water, acidified with 1 M HCl and extracted with DCM. The combined organic phases were dried over anhydrous sodium sulphate and concentrated. The product, a brown solid (2.505 g, 41%), was precipitated by addition of diethyl ether.
1 H NMR (400 MHz, DMSO-D6) δ ppm 2.80 (s, 3 H) 7.59 (d, *J*=8.33 Hz, 1 H) 7.65 (dd, *J*=8.42, 2.03 Hz, 1 H) 7.90 (d, *J*=2.02 Hz, 1 H) 7.95 (s, 1 H)
13C NMR (100 MHz, DMSO-D6) δ ppm 23.6 ($CH_3$) 109.4 (C) 115.9 (C) 121.8 (CH) 128.0 (CH) 129.4 (CH) 132.2 (CH) 132.4 (C) 133.9 (C) 135.3 (C) 150.1 (C) 155.4 (C) 161.2 (C) 166.0 (C)

Step 1C. 5-Cyano-4-(2,4-dichloro-phenyl)-6-methyl-pyridine-2-carboxylic acid methylamide (XXIa)

5-Cyano-4-(2,4-dichloro-phenyl)-6-methyl-pyridine-2-carboxylic acid (XXa) (1 eq, 3.0 mmol, 0.921 g) was dissolved in 100 mL DCM. PyBOP (1.1 eq, 3.3 mmol, 1.717 g) and triethylamine (3 eq, 9.0 mmol, 1.254 mL) were successively added followed by addition of methylamine hydrochloride (2 eq, 6.0 mmol, 0.405 g). After 3 h stirring at room temperature (reaction monitored by TLC) the solvent was removed and the product, a colorless solid (0.797 g, 83%), was purified by filtration on silica gel using PE/EA (2:1) as eluent.
1 H NMR (400 MHz, Chloroform-D) δ ppm 2.85 (s, 3 H) 3.06 (d, *J*=5.09 Hz, 3 H) 7.25 (d, *J*=8.14 Hz, 1 H) 7.39 (dd, *J*=8.27, 1.91 Hz, 1 H) 7.56 (d, *J*=2.03 Hz, 1 H) 8.03 (m, 1 H) 8.09 (s, 1 H)
13C NMR (100 MHz, Chloroform-D) δ ppm 23.9 ($CH_3$) 26.3 ($CH_3$) 111.6 (C) 115.4 (C) 120.6 (CH) 127.7 (CH) 130.2 (CH) 131.1 (CH) 133.2 (C) 133.2 (C) 136.7 (C) 151.1 (C) 151.9 (C) 161.1 (C) 163.2 (C)

Step 1 D. 5-Aminomethyl-4-(2,4-dichloro-phenyl)-6-methyl-pyridine-2-carboxylic acid methylamide (VI / XXIIa)

51 % aqueous solution of hydrazine (30 eq, 45 mmol) was added portionwise within 3 h to a suspension of 5-cyano-4-(2,4-dichloro-phenyl)-6-methyl-pyridine-2-carboxylic acid methylamide (XXIa) (1 eq, 1.5 mmol, 0.48 g) and Raney Nickel (6 mL, 50% slurry in water) in THF (60 mL). The catalyst was filtered off through celite and the solvent was removed under reduced pressure. The product, a colorless solid (0.261 g, 54%), was purified by column chromatography with DCM/MeOH (10:1) as eluent.
1H NMR (400 MHz, Methanol-D4) δ ppm 2.75 (s, 3 H) 2.96 (s, 3 H) 3.55 (d, *J*=13.99 Hz, 1 H) 3.74 (d, *J* =13.99 Hz, 1 H) 7.37 (d, *J*=8.14 Hz, 1 H) 7.48 (dd, *J*=8.39, 2.03 Hz, 1 H) 7.65 (d, *J*=2.03 Hz, 1 H) 7.66 (s, 1 H)
13C NMR (100 MHz, Methanol-D4) δ ppm 22.6 ($CH_3$) 26.4 ($CH_3$) 40.4 ($CH_2$) 121.9 (CH) 128.8 (CH) 130.5 (CH) 132.9 (CH) 134.4 (C) 136.4 (C) 137.4 (C) 137.8 (C) 148.7 (C) 148.7 (C) 159.2 (C) 167.1 (C)

EXAMPLE 2 - Synthesis of 5-aminomethyl-4-(4-methoxy-phenyl)-6-methyl-pyridine-2-carboxylic acid (5-methyl-isoxazol-3-yl)-amide hydrochloride (XXXV / XXIIb)

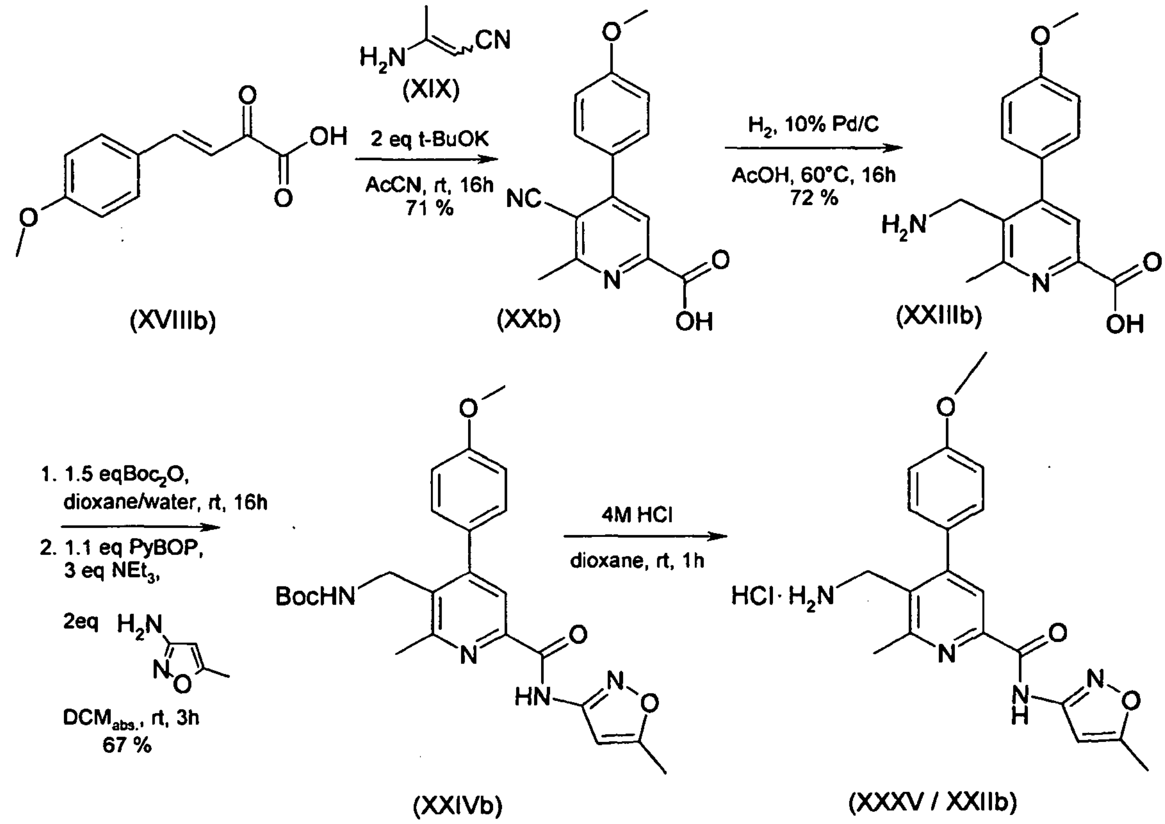


(XVIIIb) (XXb) (XXIIIb) (XXIVb) (XXXV / XXIIb)

Step 2A. 5-Cyano-4-(4-methoxy-phenyl)-6-methyl-pyridine-2-carboxylic acid (XXb)

Potassium *tert*-butoxide (2 eq, 2 mmol, 0.224 g) was added portionwise to a solution of (*E*)-2-oxo-4-(4-methoxy-phenyl)-but-3-enoic acid (XVIIIb) (1 eq, 1 mmol, 0.206 g) in acetonitrile (10 mL) stirred at room temperature. After 16 h (reaction monitored by LC/MS) the solvent was removed under reduced pressure. The crude product was taken up in water, acidified with 1 M HCl and extracted with DCM. The combined organic phases were dried over anhydrous sodium sulphate and concentrated. The product, a colorless solid (0.190 g, 71%), was purified by column chromatography with PE/EA gradient (from 4:1 to 1:1).
1 H NMR (400 MHz, DMSO-D6) δ ppm 2.77 (s, 3 H) 3.84 (s, 3 H) 7.13 (m, 2 H) 7.67 (m, 2 H) 7.92 (s, 1 H)
13C NMR (100 MHz, DMSO-D6) δ ppm 23.7 ($CH_3$) 55.4 ($CH_3$) 109.1 (C) 114.5 (CH) 116.8 (C) 121.5 (C) 127.4 (C) 130.2 (CH) 150.0 (C) 153.2 (C) 160.9 (C) 162.2 (C) 165.3 (C)

Step 2B. 5-Aminomethyl-4-(4-methoxy-phenyl)-6-methyl-pyridine-2-carboxylic acid (XXIIIb)

5-Cyano-4-(4-methoxy-phenyl)-6-methyl-pyridine-2-carboxylic acid (XXb) (1 eq, 0.25 mmol, 0.067 g) was dissolved in acetic acid (0.25 mL) and 10% Pd/C (10% m/m, 7 mg) was added. The mixture was degassed and hydrogenated overnight under 1.0 atm pressure at 60°C. The catalyst was filtered off and the solvent was removed on vacuo. The residue was taken up in water and extracted with DCM. Water was removed by lyophilization to obtain 48 mg (0.18 mmol, 72%) of the product as a colorless solid that was used in the next step without further purification.

Step 2C. [4-(4-Methoxy-phenyl)-2-methyl-6-(5-methyl-isoxazol-3-yl-carbamoyl)-pyridine-3-yl-methyl]-carbamic acid *tert*-butyl ester (XXIVb)

Di-*tert*-butyl dicarbonate (1.5 eq, 0.225 mmol, 0.049 g) was added to a solution of 5-aminomethyl-4-(4-methoxy-phenyl)-6-methyl-pyridine-2-carboxylic acid (XXIIIb) (1 eq, 0.15 mmol, 0.041 g) in dioxane (5 mL) and the mixture was stirred overnight. The solvent was removed under reduced pressure and the residue was taken up in waterfree DCM (5 mL). PyBOP (1.1 eq, 0.165 mmol, 0.085 g) and triethylamine (3 eq, 0.45 mmol, 0.063 mL) were added successively, followed by addition of 5-methyl-isoxazol-3-ylamine (2 eq, 0.30 mmol, 0.029 g). After 3 h stirring at room temperature (reaction monitored by TLC) the solvent was removed and the product was isolated as a colorless solid (0.045 g, 67%) by filtration on silica gel with PE/EA gradient (from 2:1 to 1:2).
1 H NMR (400 MHz, Chloroform-D) δ ppm 1.41 (s, 9 H) 2.42 (s, 3 H) 2.66 (s, 3 H) 3.84 (s, 3 H) 4.33 (d, *J*=4.32 Hz, 2 H) 4.61 (s, 1 H) 6.85 (s, 1 H) 6.95 (m, 2 H) 7.20 (m, 2 H) 7.88 (s, 1 H) 10.50 (s, 1 H)
13C NMR (100 MHz, Chloroform-D) δ ppm 12.7 ($CH_3$) 22.5 ($CH_3$) 28.3 ($CH_3$) 39.1 ($CH_2$) 55.3 ($CH_3$) 79.7 (C) 96.0 (CH) 114.2 (CH) 121.8 (CH) 129.7 (CH) 130.1 (C) 132.8 (C) 146.0 (C) 152.0 (C) 155.0 (C) 157.6 (C) 158.3 (C) 159.8 (C) 162.1 (C) 170.0 (C)

Step 2D. 5-Aminomethyl-4-(4-methoxy-phenyl)-6-methyl-pyridine-2-carboxylic acid (5-methyl-isoxazol-3-yl)-amide hydrochloride (XXXV / XXIIb)

[4-(4-Methoxy-phenyl)-2-methyl-6-(5-methyl-isoxazol-3-yl-carbamoyl)-pyridine-3-yl-methyl]-carbamic acid *tert*-butyl ester (XXIVb) (0.05 mmol, 0.023 mg) was dissolved in dioxane (1.0 mL) and 4 M HCl in dioxane was added (0.5 mL). After 1 h the solvent was removed under reduced pressure to yield the product, a yellow solid (0.020 g).
1 H NMR (400 MHz, Methanol-D4) δ ppm 2.43 (s, 3 H) 2.95 (s, 3 H) 3.88 (s, 3 H) 4.41 (s, 2 H) 6.73 (s, 1 H) 7.14 (m, 2 H) 7.47 (m, 2 H) 8.16 (s, 1 H)
13C NMR (100 MHz, Methanol-D4) δ ppm 12.4 ($CH_3$) 21.5 ($CH_3$) 37.7 ($CH_2$) 56.1 ($CH_3$) 97.4 (CH) 115.9 (CH) 124.7 (CH) 129.6 (C) 131.5 (CH) 146.3 (C) 158.1 (C) 158.9 (C) 159.7 (C) 161.5 (C) 162.5 (C) 171.9 (C)

EXAMPLE 3 - Synthesis of [3-aminomethyl-6-(4-chloro-phenyl)-2-methyl-pyridine-4-yl]-pyrrolidine-1-yl-methanone (XXXVI / XXXIIc)

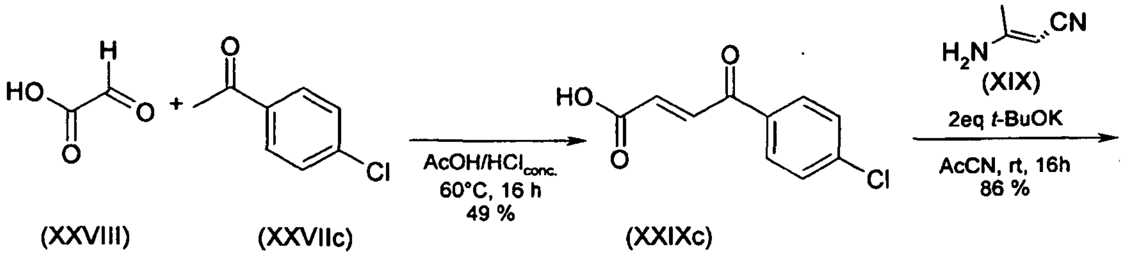


(XXVIII) (XXVIIc) (XXIXc)

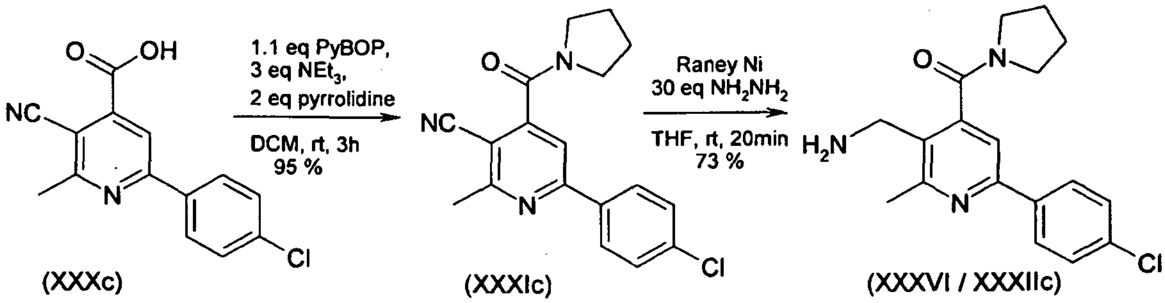


(XXXc) (XXXIc) (XXXVI / XXXIIc)

Step 3A. (*E*)-4-Oxo-4-(4-chloro-phenyl)-but-2-enoic acid (XXIXc)

Glyoxylic acid (XXVIII) (1 eq, 40 mmol, 3.68 g) was added to a solution of 4-chloro-acetophenone (XXVIIc) (1 eq, 40 mmol, 5.20 mL) in acetic acid (50 mL) and concentrated HCl (5 mL). The mixture was refluxed for 16 h. After evaporation of the solvent, the yellow residue was taken up in ethyl acetate and filtered to afford the product, a yellow solid (3.49 g, 49%) that was used in the next step without further purification.

Step 3B. 3-Cyano-6-(4-chloro-phenyl)-2-methyl-isonicotinic acid (XXXc)

Potassium *tert*-butoxide (2 eq, 2 mmol, 0.224 g) was added portionwise to a solution of (*E*)-4-oxo-4-(4-chloro-phenyl)-but-2-enoic acid (XXIXc) (1 eq, 1 mmol, 0.211 g) in acetonitrile (10 mL) stirred at room temperature. After 16 h (reaction monitored by LC/MS) the solvent was removed under reduced pressure. The crude product was taken up in water, acidified with 1 M HCl and extracted with DCM. The combined organic phases were dried over anhydrous sodium sulphate and concentrated. The product, a brown solid (0.235 g, 86%), was purified by flash chromatography with DCM/MeOH gradient (from 99:1 to 95:5).
1 H NMR (400 MHz, DMSO-D6) δ ppm 2.80 (s, 3 H) 7.58 (m, *J*=8.39 Hz, 2 H) 8.20 (m, *J*=8.65 Hz, 2 H) 8.27 (s, 1 H)
13C NMR (100 MHz, DMSO-D6) δ ppm 23.9 ($CH_3$) 105.3 (C) 116.0 (C) 117.3 (CH) 129.1 (CH) 129.1 (CH) 135.1 (C) 135.7 (CH) 143.0 (C) 157.2 (C) 162.9 (C) 164.5 (C)

Step 3C. 6-(4-Chloro-phenyl)-2-methyl-4-(pyrrolidine-1-carbonyl)-nicotinonitrile (XXXIc)

3-Cyano-6-(4-chloro-phenyl)-2-methyl-isonicotinic acid (XXXc) (1 eq, 0.5 mmol, 0.137 g) was dissolved in 20 mL DCM. PyBOP (1.1 eq, 0.55 mmol, 0.286 g) and triethylamine (3 eq, 1.5 mmol, 0.209 mL) were added successively, followed by addition of pyrrolidine (2 eq, 1.0 mmol, 0.082 mL). After 3 h stirring at room temperature (reaction monitored by TLC) the solvent was removed and the product, a colorless solid (0.155 g, 95%), was purified by filtration on silica gel using PE/EA (2: 1) as eluent.
1 H NMR (400 MHz, Chloroform-D) δ ppm 1.98 (m, 4 H) 2.84 (s, 3 H) 3.32 (m, 2 H) 3.70 (m, 2 H) 7.44 (d, *J*=8.65 Hz, 2 H) 7.60 (s, 1 H) 7.98 (d, *J*=8.65 Hz, 2 H)
13C NMR (100 MHz, Chloroform-D) δ ppm 24.1 ($CH_3$) 24.2 ($CH_2$) 26.0 ($CH_2$) 46.1 ($CH_2$) 48.3 ($CH_2$) 103.7 (C) 114.5 (CH) 115.5 (C) 128.7 (CH) 129.2 (CH) 135.4 (C) 137.0 (C) 149.8 (C) 158.7 (C) 162.5 (C) 164.2 (C)

Step 3D. [3-Aminomethyl-6-(4-chloro-phenyl)-2-methyl-pyridine-4-yl]-pyrrolidine-1-yl-methanone (XXXVI / XXXIIc)

51% aqueous solution of hydrazine (30 eq, 4.5 mmol, 0.30 mL) was added dropwise within 20 min to a suspension of 6-(4-chloro-phenyl)-2-methyl-4-(pyrrolidine-1-carbonyl)-nicotinonitrile (XXXIc) (1 eq, 0.15 mmol, 0.049 g) and Raney Nickel (1 mL, 50% slurry in water) dissolved in THF (60 mL). The catalyst was then filtered off and the solution was acidified with TFA (5 mL). The solvent was removed under reduced pressure and the product, a colorless solid (0.035 g, 73%), was purified by column chromatography with DCM/MeOH (10:1) as eluent.

1H NMR (400 MHz, Chloroform-D) δ ppm 1.99 (m, 4 H) 2.78 (s, 3 H) 3.43 (t, *J*=6.61 Hz, 2 H) 3.67 (t, *J*=6.99 Hz, 2 H) 4.18 (s, 2 H) 7.49 (m, 2 H) 7.87 (s, 1 H) 8.08 (m, 2 H)
13C NMR (100 MHz, Chloroform-D) δ ppm 22.8 ($CH_3$) 25.2 ($CH_2$) 27.1 ($CH_2$) 39.2 ($CH_2$) 47.4 ($CH_2$) 30.5 ($CH_2$) 117.0 (CH) 123.7 (C) 129.7 (CH) 130.0 (CH) 137.1 (C) 137.8 (C) 148.4 (C) 157.7 (C) 161.9 (C) 168.7 (C)

Example B - Measurement of the DPP-IV inhibitory activity, selectivity and toxicity of the inventive compounds

*In vitro* DPP-IV inhibition assay

Screening of the inventive compounds for DPP-IV inhibitory activity was performed by using a DPP-IV drug discovery kit (AK499-0001, Enzo Life Sciences), wherein the inhibition of human recombinant DPP-IV activity was measured by following the absorbance increase upon cleavage of the chromogenic substrate Gly-Pro-pNA (pNA is p-nitroaniline) as described in the instruction booklet of the supplier.

The assay was performed in 96-well plates at 37°C. First the enzyme was preincubated with the test compounds for 10 min. Therefore each well was filled with 0.18 mU of enzyme and variable concentrations of the inhibitor. Each well was filled up with Tris buffer (50 mM, pH 7.5) to a final volume of 200 μL. For dilutions of all test compounds DMSO was used. The final DMSO concentration in the test did not exceed 1%. The final substrate concentration was chosen around $K_m$ value obtained under the assay conditions, as 50 μM.

After preincubation period the substrate was added and the reaction was followed by continuous absorbance measurement at 405 nm using a MRX Revelation plate reader (Dynex Technologies).

The assay was repeated 3 times for each test compound. $IC_{50}$ values were calculated after plotting the data for up to eight inhibitor concentrations by mean.

By measuring the rate of hydrolysis of Gly-Pro-pNA at four different concentrations of substrate and inhibitor the type of inhibition was determined. To calculate the initial rate of pNA formation the absorption at 405 nm was converted to micromoles of pNA using a standard calibration curve. For each concentration of inhibitor the initial rates were used for Lineweaver-Burk plot.

It could be found that the described inventive compounds act as competitive inhibitors. The inhibition constant $K_i$ was obtained from the expression:

$$K_i = i / [(K_p/K_m) - 1],$$

wherein $K_m$ is concentration of substrate that leads to half-maximal velocity, and Kp is the effective Michaelis constant in presence of the inhibitor at concentration i.

*In vitro* DPP-8 inhibition assay

The screening of the compounds for DPP-8 inhibitory activity was performed using human recombinant DPP-8, supplied by Enzo Life Sciences. The substrate used for the screening was also Gly-Pro-pNA at a concentration chosen around $K_m$ value obtained under the assay conditions, 300 μM. The other parameters and conditions of the assay were identical as previously described for DPP-IV.

Tab.1: Inhibitory concentration 50% ($IC_{50}$) of some of the tested inventive compounds (nd - not determined).

| tested compound | $IC_{50}$ [μM] | |
|---|---|---|
| | DPP-IV | DPP-8 |
| (V) | 0.016 | 33 |
| (VI) | 0.011 (0.006) | 39 |
| (VII) | 0.010 | 66 |
| (VIII) | 0.044 | 25 |
| (IX) | 0.080 | 106 |
| (X) | 0.686 | 137 |
| (XI) | 0.674 | 179 |
| (XII) | 0.937 | 182 |

(continued)

| tested compound | $IC_{50}$ [μM] | |
|---|---|---|
| | DPP-IV | DPP-8 |
| (XIII) | <10 | nd |
| (XIV) | <10 | nd |
| regioisomer (XV) | <10 | nd |

As shown above, the compounds of the present invention have a superior DPP-IV inhibitory activity in nanomolar range. The $IC_{50}$ levels found are in the same range like the activities of DPP-IV inhibitors actually on the market. But in addition our inventive compounds show a very high selectivity to DPP-IV. For inhibition of the activity of the structurally similar DPP-8 enzyme around 3 orders of magnitude higher concentrations are necessary.

Toxicity of the inventive compounds

The toxicity of the compounds (V) to (XV) was investigated with HeLa cells (Human cervix carcinoma [ATCC Cat.No. CCL-2]). During this experiments no toxic effects were observed on the cell line at a test compound concentration of 10 μM.

## Claims

1. A compound of formula (I) with the general structure

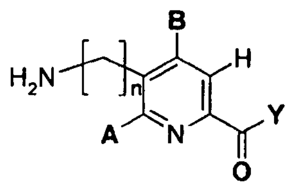

(I)

wherein

n is 1 or 2;
A is alkyl consisting of 1 to 6 C-atoms;
B is aryl, which may optionally be substituted with one to five substituents selected from the group consisting of hydrogen (H), halogen, $CF_3$, alkyl, and alkoxy;
Y is selected from the group consisting of hydroxy, alkoxy, and $-NR^3R^4$, wherein
$R^3$ and $R^4$ are the same or different and are

(i) each independently selected from the group consisting of hydrogen (H), alkyl, cycloalkyl or saturated and unsaturated heterocycloalkyl, wherein either functional group may optionally be substituted with one to three substituents selected from the group consisting of hydrogen (H), alkyl, alkylester, alkylamino, heterocycloalkyl, heterocycloalkylamino, substituted or unsubstituted aryl, amino, and cyano;
(ii) $R^3$ and $R^4$ in $-NR^3R^4$ form together with the adjacent nitrogen atom a 3- to 6-membered saturated or partially unsaturated heterocycloalkyl ring system, which may optionally be substituted with one to three substituents selected from the group consisting of hydrogen (H), alkyl, alkylester, alkyl-amino, heterocycloalkyl, heterocycloalkylamino, substituted or unsubstituted aryl, amino, and cyano;

as well as a pharmaceutically acceptable salt, a solvate, and all tautomers, stereoisomers or mixtures thereof.

2. A compound according to claim 1 selected from the group of

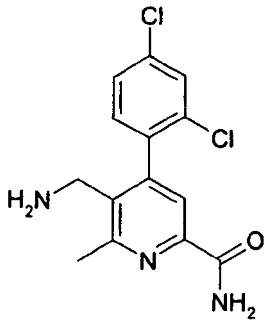
(V)

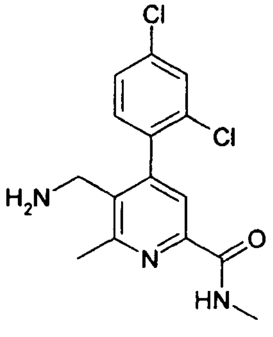
(VI)

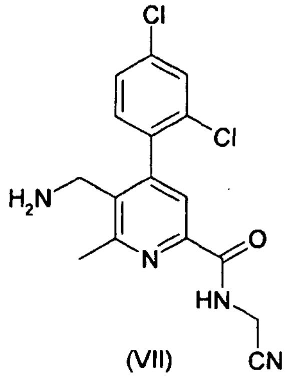
(VII)

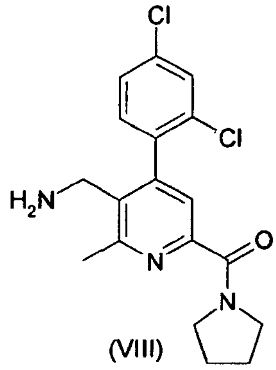
(VIII)

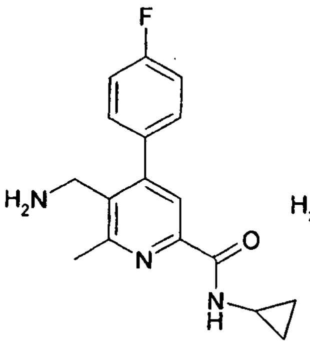
(IX)

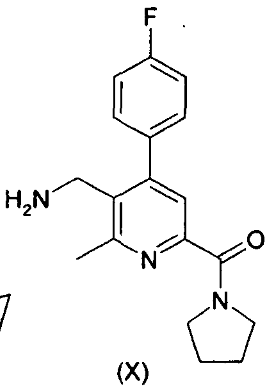
(X)

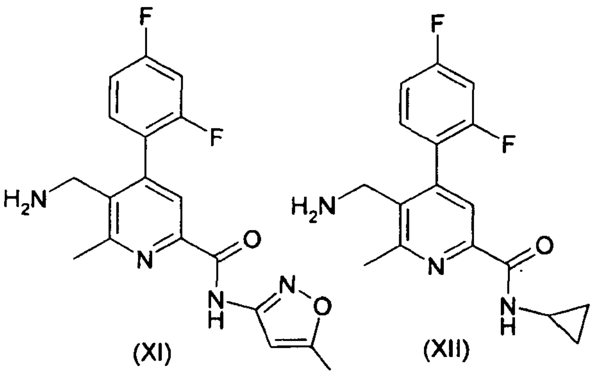
(XI)

(XII)

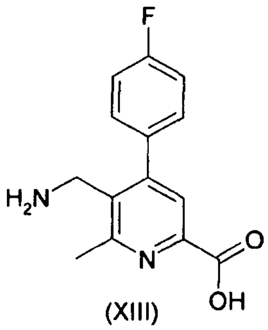
(XIII)

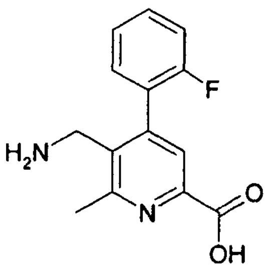
(XIV)

as well as in each case a pharmaceutically acceptable salt, a solvate, and all tautomers, stereoisomers or mixtures thereof.

3. A method for synthesis of a compound according to formula (I) as defined in claims 1 and 2, which comprises aldol condensation of aromatic aldehydes with pyruvic acid, cyclisation to substituted pyridine-2-carboxylic acid, amidation of the carboxylic group and catalytic hydrogenation of the nitrile or alternatively catalytic hydrogenation of the nitrile group before the amidation under Boc-protection of the amine group.

4. A pharmaceutical composition comprising a therapeutically effective amount of a compound as defined in claims 1 and 2 alone or in combination, and optionally one or more pharmaceutically active agents, materials and adjuvants, and/or one ore more pharmaceutically acceptable carriers, excipients and diluents.

5. A pharmaceutical composition as defined in claim 4, further comprising at least one therapeutic agent selected from the group consisting of, but not limited to, other DPP-IV inhibitors, other anti-diabetic agents, anti-hyperglycemic agents, hypolipidemic/ lipid-lowering agents, anti-obesity agents, appetite suppressants, other therapeutic agents such as anti-hypertensive agents, anti-platelet agents, anti-arteriosclerosis agents, therapeutic agents of osteoporosis, diuretic agents, agents for improving erectile dysfunction, therapeutic agents for incontinentia or pollakiuria, therapeutic agents for dysurea, chemotherapeutic agents, immunotherapeutic agents, anti-thrombotic agents, anti-dementia, agonists for peroxisome proliferator-activator receptors, and the like, and other pharmaceutically active materials, agents or adjuvants.

6. Use of a compound as defined in claims 1 and 2, for the production of a diagnostic kit for clinical applications and scientific research.

7. Use of a compound as defined in claims 1 and 2, for the production of an agent for the prophylaxis, treatment or delaying of the progression or onset of conditions, diseases, and maladies mediated by DPP-IV, such as diabetes, especially non-insulin dependent diabetes mellitus, more especially type II diabetes, and related diseases such as type I diabetes, impaired glucose tolerance, impaired fasting plasma glucose, insulin resistance, insulin secretagogue, cachexia, hyperglycemia, hyperinsulinemia, hypercholesterolemia, fatty liver, elevated blood levels of fatty acids or glycerol, increasing high density lipoprotein levels, obesity, hyperlipidemia including hypertriglyceridemia, Syndrome X, dyslipidemia, acidosis, ketosis; including but not limited to complications of diabetes such as retinopathy, neuropathy, nephropathy, erectile dysfunction, cataracts, delayed wound healing, arthritis, calcitonin-osteoporosis, ischemia, arteriosclerosis, polycystic ovary syndrome, kidney disease, muscular dystrophy, myocardial infarction, angina pectoris, cerebrovascular accident, Alzheimer's disease, Parkinson's syndrome, anxiety, dementia, schizophrenia, metabolic syndrome, hyperinsulinemia-induced sensory disorder, tumor, irritable bowel syndrome, acute or chronic diarrhea, inflammatory diseases, small intestine mucous membrane trauma, malabsorption, testis function disorder, visceral obesity syndrome, hypertension, various immunomodulatory diseases; and further conditions of impaired glucose tolerance; as well as to improve pancreatic ß cell function, to support regeneration of pancreatic ß cells, to produce a sedative or anxiolytic effect, to attenuate post-surgical catabolic changes and hormonal responses to stress, to reduce mortality and morbidity after myocardial infarction, and to effect a cosmetically beneficial loss of body weight.

8. Use of a compound as defined in claims 1 and 2, for the production of a peptidase inhibiting agent, medicament or pharmaceutical composition, wherein the peptidase is DPP-IV, for preventing, treating or delaying of the progression or onset of conditions, diseases, and maladies mediated by DPP-IV, such as diabetes, especially non-insulin dependent diabetes mellitus, more especially type II diabetes, and related diseases such as type I diabetes, impaired glucose tolerance, impaired fasting plasma glucose, insulin resistance, insulin secretagogue, cachexia, hyperglycemia, hyperinsulinemia, hypercholesterolemia, fatty liver, elevated blood levels of fatty acids or glycerol, increasing high density lipoprotein levels, obesity, hyperlipidemia including hypertriglyceridemia, Syndrome X, dyslipidemia, acidosis, ketosis; including but not limited to complications of diabetes such as retinopathy, neuropathy, nephropathy, erectile dysfunction, cataracts, delayed wound healing, arthritis, calcitonin-osteoporosis, ischemia, arteriosclerosis, polycystic ovary syndrome, kidney disease, muscular dystrophy, myocardial infarction, angina pectoris, cerebrovascular accident, Alzheimer's disease, Parkinson's syndrome, anxiety, dementia, schizophrenia, metabolic syndrome, hyperinsulinemia-induced sensory disorder, tumor, irritable bowel syndrome, acute or chronic diarrhea, inflammatory diseases, small intestine mucous membrane trauma, malabsorption, testis function disorder, visceral obesity syndrome, hypertension, various immunomodulatory diseases; and further conditions of impaired glucose tolerance; as well as to improve pancreatic ß cell function, to support regeneration of pancreatic ß cells, to produce a sedative or anxiolytic effect, to attenuate post-surgical catabolic changes and hormonal responses to stress, to reduce mortality and morbidity after myocardial infarction, and to effect a cosmetically beneficial loss of body weight, in a mammal.

## Patentansprüche

1. Eine Verbindung gemäß Formel (I) mit der allgemeinen Struktur

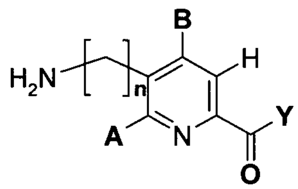

(I)

worin

n 1 oder 2 ist;
A für eine Alkyl-Gruppe, bestehend aus ein bis sechs C-Atomen, steht;
B für eine Aryl-Gruppe steht, die optional mit ein bis fünf Substituenten aus der Gruppe Wasserstoff (H), Halogen, $CF_3$, Alkyl und Alkoxy, substituiert sein kann;
Y aus der Gruppe Hydroxy, Alkoxy und $-NR^3R^4$ ausgewählt ist, wobei
$R^3$ und $R^4$ gleich oder verschieden sein können und

(i) jedes unabhängig aus der Gruppe Wasserstoff (H), Alkyl, Cycloalkyl und gesättigtes oder ungesättigtes Heterocycloalkyl ausgewählt ist, wobei jede funktionelle Gruppe optional mit ein bis drei Substituenten aus der Gruppe Wasserstoff (H), Alkyl, Alkylester, Alkylamino, Heterocycloalkyl, Heterocycloalkylamino, substituiertes oder unsubstituiertes Aryl, Amino und Cyano, substituiert sein kann;
(ii) $R^3$ und $R^4$ in $-NR^3R^4$ zusammen mit dem benachbarten Stickstoff-Atom ein 3- bis 6-gliedriges gesättigtes oder teilweise ungesättigtes heterocyclisches Ringsystem bilden, welches optional mit ein bis drei Substituenten aus der Gruppe Wasserstoff (H), Alkyl, Alkylester, Alkylamino, Heterocycloalkyl, Heterocycloalkylamino, substituiertes oder unsubstituiertes Aryl, Amino und Cyano, substituiert sein kann;

sowie ein pharmazeutisch akzeptables Salz, ein Solvat und alle Tautomere, Stereoisomere oder Mischungen hiervon.

2. Eine Verbindung nach Anspruch 1, ausgewählt aus der Gruppe

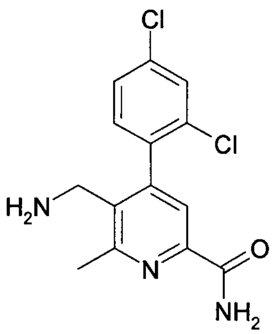
(V)

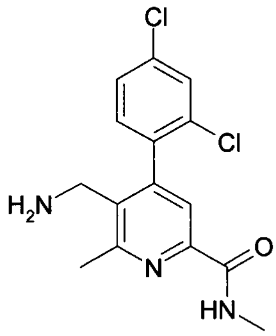
(VI)

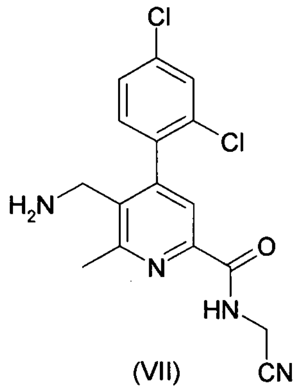
(VII)

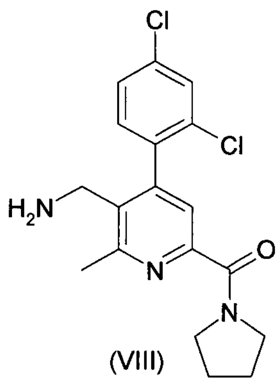
(VIII)

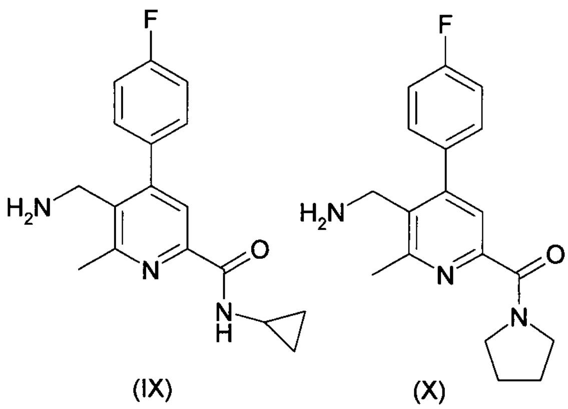
(IX) (X)

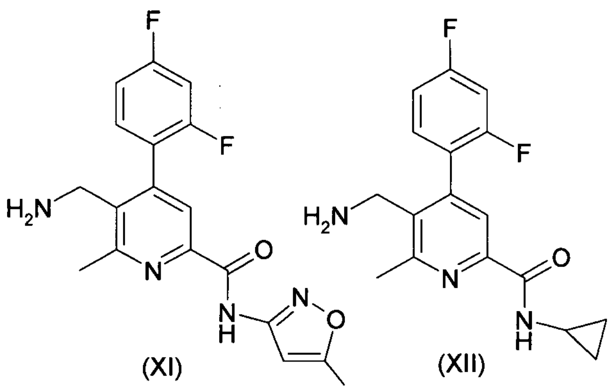
(XI) (XII)

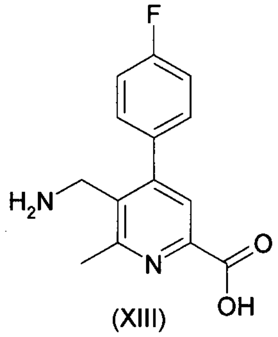
(XIII)

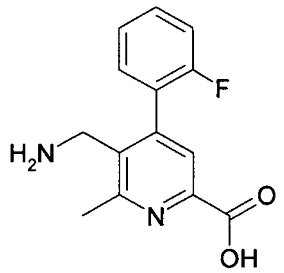
(XIV)

sowie in jedem Fall ein pharmazeutisch akzeptables Salz, ein Solvat und alle Tautomere, Stereoisomere oder Mischungen hiervon.

3. Verfahren zur Herstellung einer Verbindung gemäß Formel (I) nach den Ansprüchen 1 und 2, das die Schritte Aldol-Kondensation eines aromatischen Aldehydes mit Brenztraubensäure, Zyklisierung zu einer substituierten Pyridin-2-Carbonsäure, Amidierung der Carboxy-Gruppe und katalytische Hydrierung des Nitrils oder alternativ zuerst die katalytische Hydrierung der Nitril-Gruppe vorderAmidierung der Carboxy-Gruppe bei Boc-geschützter Amino-Gruppe, umfasst.

4. Pharmazeutische Zusammensetzung, enthaltend eine therapeutisch effektive Menge einer Verbindung nach den Ansprüchen 1 und 2 allein oder in Kombination, und optional einen oder mehrere pharmazeutisch aktive Stoffe, Materialien und Adjuvantien und/oder einen oder mehrere pharmazeutisch akzeptable Träger, Hilfs- und Zusatzstoffe, Verdünnungsmittel und Füllstoffe.

5. Pharmazeutische Zusammensetzung nach Anspruch 4, weiter enthaltend mindestens einen therapeutischen Wirkstoff, ausgewählt aus der folgenden Gruppe, aber nicht beschränkt darauf: andere DPP-IV Inhibitoren, andere Anti-Diabetika, Anti-Hyperglykämika, Hypolipidämika / Lipidsenker, Anti-Adipositas-Mittel, Appetitzügler, andere therapeutische Mittel, wie z.B. Blutdrucksenker, Thrombozytenaggregationshemmer, Anti-Arteriosklerotika, Anti-Osteoporotika, Diuretika, Mittel zur Besserung von Erektionsstörungen, therapeutische Mittel gegen Inkontinenz oder Pollakisurie, therapeutische Mittel gegen Dysurie, Chemotherapeutika, Immunotherapeutika, Anti-Thrombotika, Anti-Dementiva, Agonisten für Peroxisom-Proliferator-aktivierte Rezeptoren und dergleichen, und andere pharmazeutisch aktive Materialien, Wirkstoffe oder Adjuvantien.

6. Verwendung einer Verbindung nach den Ansprüchen 1 und 2 für die Herstellung eines Diagnose-Kits für klinische Anwendungen und für die wissenschaftliche Forschung.

7. Verwendung einer Verbindung nach den Ansprüchen 1 und 2 für die Herstellung eines Mittels zur Prophylaxe, Behandlung oder Verzögerung des Verlaufes oder des Ausbrechens von Symptomen, Krankheiten und Leiden, die mit DPP-IV in Verbindung stehen, wie z.B. von Diabetes, speziell insulin-unabhängigem Diabetes mellitus, insbesondere Typ-2-Diabetes; und von damit verbundenen Krankheiten, wie z.B. Typ-1-Diabetes, gestörter Glucosetoleranz, erhöhtem Nüchtern-Plasmaglucose-Spiegel, Insulin-Resistenz, verminderter Insulin-Sekretion, Kachexie, Hyperglykämie, Hyperinsulinämie, Hypercholesterinämie, Fettleber, erhöhten Fettsäure- und Glycerin-Blutspiegeln, steigenden High-Density-Lipoprotein-Spiegeln, Adipositas, Hyperlipidämie einschließlich Hypertriglyceridämie, Syndrom X, Dyslipidämie, Azidose, Ketose; einschließlich von, aber nicht beschränkt auf, Komplikationen von Diabetes, wie z.B. Retinopathie, Neuropathie, Nephropathie, Erektionsstörungen, Grauem Star, verzögerter Wundheilung, Arthritis, Calcitonin-abhängiger Osteoporose, Ischämie, Arteriosklerose, polyzystischem Ovarialsyndrom, Nierenerkrankungen, Muskeldystrophie, Herzinfarkt, Angina pectoris, Schlaganfall, Alzheimer-Krankheit, Parkinson-Syndrom, Angstzuständen, Demenz, Schizophrenie, metabolischem Syndrom, Hyperinsulinämie-bedingten sensorischen Störungen, Tumoren, Reizdarmsyndrom, akutem oder chronischem Durchfall, entzündlichen Erkrankungen, Schädigungen der Dünndarmschleimhaut, Malabsorption, Hoden-Funktionsstörungen, viszeraler Adipositas, Hypertonie, verschiedenen immunmodulatorischen Krankheiten; und von anderen durch eine gestörte Glucosetoleranz bedingten Symptomen; sowie zur Verbesserung der Funktion und zur Unterstützung der Regeneration der ß-Zellen der Bauchspeicheldrüse, zur Erzielung eines beruhigenden und angstlösenden Effektes, zurAbmilderung von post-operativen Abbau-Prozessen und hormonellen Stress-Reaktionen, zur Verminderung der Sterblichkeit und Morbidität nach einem Herzinfarkt und zur Erzielung einer kosmetisch vorteilhaften Reduzierung des Körpergewichtes.

**8.** Verwendung einer Verbindung nach den Ansprüchen 1 und 2 für die Herstellung eines/einer Peptidase-inhibierenden Stoffes, Medikaments oder pharmazeutischen Zusammensetzung, wobei die Peptidase DPP-IV ist, zum Vorbeugen, Behandeln oder Verzögern des Verlaufes oder des Ausbrechens von Symptomen, Krankheiten und Leiden, die mit DPP-IV in Verbindung stehen, wie z.B. von Diabetes, speziell insulin-unabhängigem Diabetes mellitus, insbesondere Typ-2-Diabetes; und von damit verbundenen Krankheiten, wie z.B. Typ-1-Diabetes, gestörter Glucosetoleranz, erhöhtem Nüchtern-Plasmaglucose-Spiegel, Insulin-Resistenz, verminderter Insulin-Sekretion, Kachexie, Hyperglykämie, Hyperinsulinämie, Hypercholesterinämie, Fettleber, erhöhten Fettsäure- und Glycerin-Blutspiegeln, steigenden High-Density-Lipoprotein-Spiegeln, Adipositas, Hyperlipidämie einschließlich Hypertriglyceridämie, Syndrom X, Dyslipidämie, Azidose, Ketose; einschließlich von, aber nicht beschränkt auf, Komplikationen von Diabetes, wie z.B. Retinopathie, Neuropathie, Nephropathie, Erektionsstörungen, Grauem Star, verzögerter Wundheilung, Arthritis, Calcitonin-abhängiger Osteoporose, Ischämie, Arteriosklerose, polyzystischem Ovarialsyndrom, Nierenerkrankungen, Muskeldystrophie, Herzinfarkt, Angina pectoris, Schlaganfall, Alzheimer-Krankheit, Parkinson-Syndrom, Angstzuständen, Demenz, Schizophrenie, metabolischem Syndrom, Hyperinsulinämie-bedingten sensorischen Störungen, Tumoren, Reizdarmsyndrom, akutem oder chronischem Durchfall, entzündlichen Erkrankungen, Schädigungen der Dünndarmschleimhaut, Malabsorption, Hoden-Funktionsstörungen, viszeraler Adipositas, Hypertonie, verschiedenen immunmodulatorischen Krankheiten; und von anderen durch eine gestörte Glucosetoleranz bedingten Symptomen; sowie zur Verbesserung der Funktion und zur Unterstützung der Regeneration der ß-Zellen der Bauchspeicheldrüse, zur Erzielung eines beruhigenden und angstlösenden Effektes, zur Abmilderung von postoperativen Abbau-Prozessen und hormonellen Stress-Reaktionen, zur Verminderung der Sterblichkeit und Morbidität nach einem Herzinfarkt und zur Erzielung einer kosmetisch vorteilhaften Reduzierung des Körpergewichtes, bei einem Säuger.

**Revendications**

**1.** Un composé de formule (I) avec la structure générale

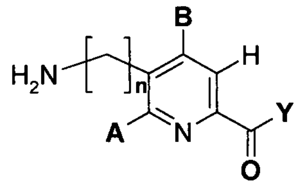

(I)

où

n est 1 ou 2;
A est un groupe alkyle comprenant de un à six atomes de carbone;
B est un groupe aryle, qui peut éventuellement être substitué par un à cinq substituants, choisis dans le groupe constitués par l'hydrogène (H), un halogène, $CF_3$, un groupe alkyle et un groupe alkoxy;
Y est choisi dans le groupe constitué par hydroxy, alkoxy et $-NR^3R^4$ dans lequel
$R^3$ et $R^4$ sont identiques ou différents et sont

(i) chacun indépendamment choisi parmi les groupes constitués par l'hydrogène (H), un groupe alkyle, cycloalkyle et hétérocycloalkyle saturé ou insaturé dans lesquels d'autres groupes fonctionnels peuvent évntuellementt être substitués par un à trois substituants substituants, choisis dans le groupe constitué par l'hydrogène (H), les alkyles, les alkylesters, les alkylamino, les hétérocycloalkyle, les hétérocycloalkylamino ou encore les aryles, les amino et les cyano substitués ou non;
(ii) $R^3$ et $R^4$ dans $-NR^3R^4$ forment ensemble avec l'atome d'azote adjacent un akyle hétérocyclique composé de trois à six atomes, saturé ou partiellement insaturé, qui peut éventuellement être substitué de un jusqu'à trois substituants, choisi dans le groupe constitué par l'hydrogène (H), les alkyles, les alkylesters, les alkylamino, les hétérocycloalkyle, les hétérocyclo-alkylamino ou encore les aryles, les amino et les cyano substitués ou non;

aussi bien qu'un sel pharmaceutiquement acceptable, un solvate et tous les tautomères, stéréo-isomères ou leurs mélanges.

**2.** Un composé selon la revendication 1, choisi dans le groupe

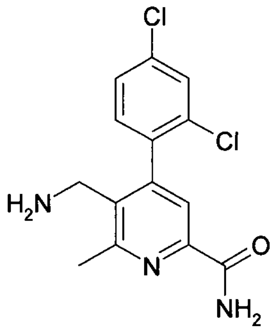

(V)

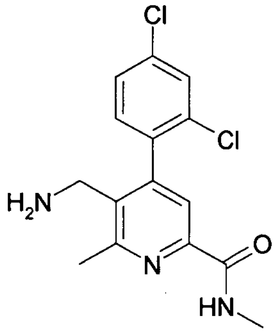

(VI)

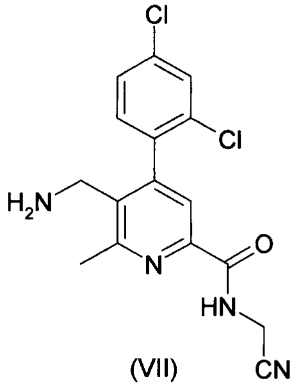

(VII)

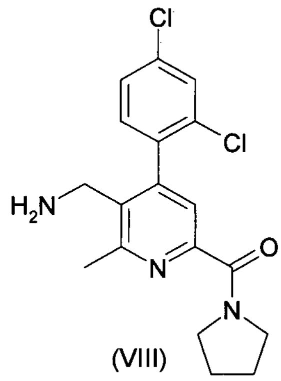

(VIII)

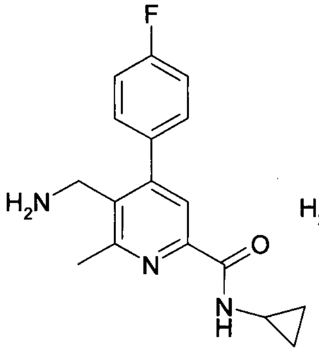

(IX)

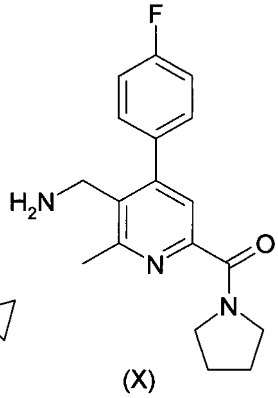

(X)

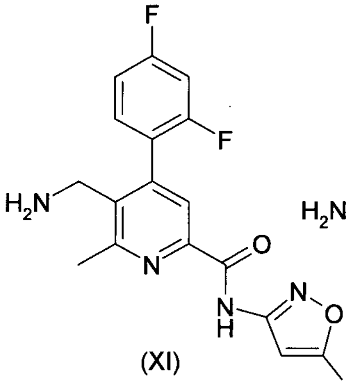

(XI)

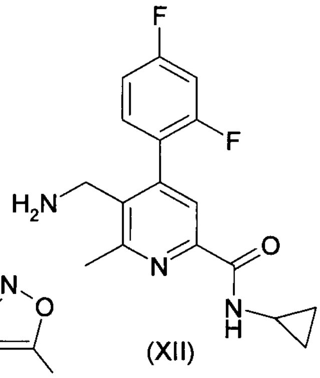

(XII)

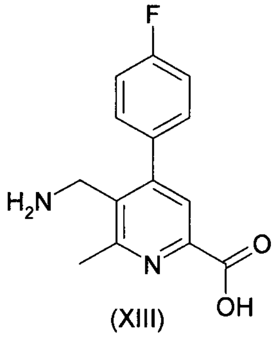

(XIII)

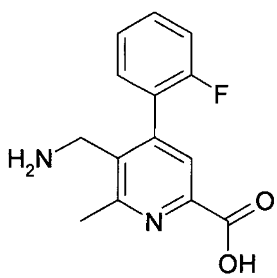

(XIV)

aussi bien pour chaque cas qu'un sel acceptable dans la pharmaceutique, un solvate et tous les tautomères, stéréo-isomères ou leurs mélanges.

**3.** Procédé de synthèse d'un composé selon la formule (I) tel que défini dans les revendications 1 et 2 qui comprend la condensation aldolique d'aldéhydes aromatiques avec des acides pyruviques, la cyclisation en dérivés acides 2-pyridine carboxiliques substitués, l'amidation de groupes carboxyliques et l'hydrogénation catalytique de groupes nitriles ou de manière alternative, l'hydrogénation catalytique de groupes nitriles avant l'amidation du groupe carboxylique de groupes amines protégés par Boc.

**4.** Composition pharmaceutique comprenant une quantité thérapeutiquement efficace d'un composé tel que défini dans les revendications 1 et 2 seul ou en combinaison, et éventuellement un ou plusieurs agents actifs, des matériaux

et des adjuvants et/ou un ou plusieurs supports pharmaceutiquement acceptables, des excipients et des diluants.

**5.** Une composition pharmaceutique telle que définie dans la revendication 4, comprenant en outre au moins un agent thérapeutique, choisi dans le groupe constitué par, mais sans s'y limiter, d'autres inhibiteurs de DPP-IV, d'agents anti-diabète, d'agents anti-hyperglycémiques, d'agents hypolipidémiants, d'agents anti-obésité, des inhibiteurs de l'appétit, d'autres agents thérapeutiques tels que les agents anti-hypertensifs, d'agents anti-plaquettaires, d'agents anti-artériosclérose, d'agents thérapeutiques pour l'ostéoporose, des agents diurétiques, d'agents pour améliorer les problèmes érectiles, d'agents thérapeutiques pour incontinence ou pollakiurie, d'agents thérapeutiques pour la dysurie, d'agents chimio-thérapeutiques, d'agents immunothérapeutiques, d'agents anti-thrombotiques, d'agents anti-démence, des agonistes du récepteur activés par les proliférateurs des péroxisomes et autres analogues, et d'autres substances, agents ou adjuvants pharmaceutiquement actifs.

**6.** L'utilisation d'un composé tel que défini dans les revendications 1 et 2, pour la production de kits diagnostiques pour des applications cliniques ou la recherche scientifique.

**7.** L'utilisation d'un composé tel que défini dans les revendications 1 et 2, pour la production d'un agent pour la prophylaxie, le traitement ou le retardement de la progression ou l'apparition de conditions, maladies, et des maladies régulées par DPP-IV, telles que le diabète et plus particulièrement le diabète non insulino-dépendant, plus spécialement les diabètes de type II; et tout autres maladies connexes comme le diabète de type I, l'intolérance au glucose, l'altération du glucose plasmatique à jeun, la résistance à l'insuline, problème de sécrétion d'insuline, la cachexie, l'hyperglycémie, l'hyper-insulinémie, hypercholestérolémie, la stéatose hépatique, taux sanguins élevés d'acides gras ou de glycérol, l'augmentation des taux de lipoprotéines de haute densité, l'obésité, l'hyperlipidémie y compris l'hypertriglycéridémie, le syndrome X, la dyslipidémie, l'acidose, la cétose; y compris, mais sans s'y limiter, les complications du diabète telles que la rétinopathie, la neuropathie, la néphropathie, la dysfonction érectile, les cataractes, le retard de cicatrisation, l'arthrite, la calcitonine-ostéoporose, l'ischémie, l'artériosclérose, le syndrome d'ovaire poly-kystique, des maladies rénales, dystrophie musculaire, l'infarctus du myocarde, l'angine de poitrine, d'un accident vasculaire cérébral, la maladie d'Alzheimer, la maladie de Parkinson, l'anxiété, la démence, la schizophrénie, le syndrome métabolique, troubles sensoriels induits par une hyper-insulinémie, une tumeur, le syndrome du côlon irritable, la diarrhée aiguë ou chronique, les maladies inflammatoires, traumatisme de la membrane de la muqueuse de l'intestin grêle, de la malabsorption, un trouble de la fonction testiculaire, le syndrome de l'obésité viscérale, de l'hypertension, diverses maladies immuno-modulatrices; et d'autres conditions dues à l'intolérance au glucose; ainsi que pour améliorer la fonction des cellules ß du pancréas, de soutenir la régénération des cellules ß pancréatiques, pour produire un effet sédatif ou anxiolytique, pour atténuer les changements cataboliques post-chirurgicaux et les réponses hormonales au stress, pour réduire la mortalité et la morbidité après un infarctus du myocarde, et pour effectuer une perte bénéfique de poids corporel (effet cosmétique).

**8.** Utilisation d'un composé tel que défini dans les revendications 1 et 2, pour la production d' agents inhibiteurs de peptidase, de médicaments ou de compositions pharmaceutiques dans lesquels la peptidase est la DPP- IV, pour prévenir, traiter ou retarder la progression ou l'apparition de conditions, de maladies et des maladies régulées par DPP-IV, telles que le diabète et plus particulièrement le diabète non insulino-dépendant, plus spécialement les diabètes de type II; et tout autres maladies connexes comme le diabète de type I, l'intolérance au glucose, l'altération du glucose plasmatique à jeun, la résistance à l'insuline, problème de sécrétion d'insuline, la cachexie, l'hyperglycémie, l'hyper-insulinémie, hypercholestérolémie, la stéatose hépatique, taux sanguins élevés d'acides gras ou de glycérol, l'augmentation des taux de lipoprotéines de haute densité, l'obésité, l'hyperlipidémie y compris l'hypertriglycéridémie, le syndrome X, la dyslipidémie, l'acidose, la cétose; y compris, mais sans s'y limiter, les complications du diabète telles que la rétinopathie, la neuropathie, la néphropathie, la dysfonction érectile, les cataractes, le retard de cicatrisation, l'arthrite, la calcitonine-ostéoporose, l'ischémie, l'artériosclérose, le syndrome d'ovaire poly-kystique, des maladies rénales, dystrophie musculaire, l'infarctus du myocarde, l'angine de poitrine, d'un accident vasculaire cérébral, la maladie d'Alzheimer, la maladie de Parkinson, l'anxiété, la démence, la schizophrénie, le syndrome métabolique, troubles sensoriels induits par une hyper-insulinémie, une tumeur, le syndrome du côlon irritable, la diarrhée aiguë ou chronique, les maladies inflammatoires, traumatisme de la membrane de la muqueuse de l'intestin grêle, de la malabsorption, un trouble de la fonction testiculaire, le syndrome de l'obésité viscérale, de l'hypertension, diverses maladies immuno-modulatrices; et d'autres conitions dues à l'intolérance au glucose; ainsi que pour améliorer la fonction des cellules ß du pancréas, de soutenir la régénération des cellules ß pancréatiques, pour produire un effet sédatif ou anxiolytique, pour atténuer les changements cataboliques post-chirurgicaux et les réponses hormonales au stress, pour réduire la mortalité et la morbidité après un infarctus du myocarde, et pour effectuer une perte bénéfique de poids corporel (effet cosmétique) chez le mammifère.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5424286 A
- WO 2003004498 A
- WO 2000034241 A
- WO 2001068603 A
- WO 2005095381 A
- US 7238724 B
- WO 2003068757 A
- WO 2005042488 A
- WO 2006090915 A
- WO 2006127287 A
- WO 2008064107 A

### Non-patent literature cited in the description


- **V.K.HOPSU-HAV ; G.G.GLENNER.** *Histochem.,* 1966, vol. 7, 197-201
- **D.J.DRUCKER.** *Diabetes Care,* 2007, vol. 30 (6), 1335-1343
- **S.LOREY et al.** *J. Biol. Chem.,* 2002, vol. 277 (36), 33170-33177
- **B.D.GREEN et al.** *Diab.Vasc.Dis.Res.,* 2006, vol. 3 (3), 159-165
- **I.QUESADA et al.** *J.Endocrin,* 2008, vol. 199, 5-19
- **A.H.BARNETT.** *Clin.Endocrin.,* 2009, vol. 70, 343-353
- **B.KUHN et al.** *Current Topics in Medicinal Chemistry,* 2007, vol. 7, 609-619
- **S.H.HAVALE ; M.PAL.** *Bioorg.Med.Chem.,* 2009, vol. 17, 1783-1802
- **B.T.SRINIVASAN et al.** *Postgrad.Med.J.,* 2008, vol. 84, 524-531
- **H.FUKUSHIMA et al.** *Bioorg.Med.Chem,* 2008, vol. 16, 4093-4106
- **H.FUKUSHIMA et al.** *Chem.Pharm.Bull.,* 2008, vol. 56 (8), 1110-1117
- **T.LÜBBERS et al.** *Bioorg.Med.Chem.Lett.,* 2007, vol. 17, 2966-2970
- **J.U.PETERS et al.** *Bioorg. Med. Chem.Lett.,* 2004, vol. 14, 3575-3578
- **T.E.HUGHES et al.** *Biochemistry,* 1999, vol. 38, 11597-11603
- **D.J.MADAR et al.** *J.Med.Chem.,* 2006, vol. 49, 6416-6420
- **G.R.LANKAS et al.** *Diabetes,* 2007, vol. 56, A138
- **J.SCHADE et al.** *J.Histochem.Cytochem.,* 2008, vol. 56, 147-155
- **P.VANDERVEKEN et al.** *Current Topics in Med.Chem.,* 2007, vol. 7, 621-635
- **P.VAN DER VEKEN et al.** *Bioorg.Med.Chem. Lett.,* 2008, vol. 18, 4154-4158
- **C.W.CHIA ; J.M.EGAN.** *J.Clin.Endocrinol.Metab.,* 2008, vol. 93 (10), 3703-3716
- **S.LOREY et al.** *Eur.J.Biochem.,* 2003, vol. 270, 2147-2156
- **Y.-C.WU et al.** *J.Org.Chem.,* 2006, vol. 71, 6592
- **R.MESSER et al.** *J.Org.Chem.,* 2004, vol. 69, 8558